(19)

Europäisches
Patentamt
European
Patent Office
Office européen
des brevets

(11) **EP 4 593 022 B1**

(12) **EUROPEAN PATENT SPECIFICATION**

(45) Date of publication and mention
of the grant of the patent:
**29.07.2026 Bulletin 2026/31**

(21) Application number: **23904788.9**

(22) Date of filing: **12.12.2023**

(51) International Patent Classification (IPC):
*G16H 20/30* (2018.01)     *G16H 10/60* (2018.01)
*G16H 50/20* (2018.01)     *G16H 50/30* (2018.01)
*A61B 5/11* (2006.01)     *G06V 40/20* (2022.01)
*G06N 20/00* (2019.01)     *A61B 5/00* (2006.01)

(52) Cooperative Patent Classification (CPC):
**G06N 20/00; A61B 5/1116; A61B 5/1128;
A61B 5/4561; A61B 5/7267; G06N 3/045;
G06N 3/08; G06V 10/82; G06V 20/64;
G06V 40/103; G16H 20/30; G16H 40/67;
G16H 50/20; G16H 50/30;** A61B 5/744

(86) International application number:
**PCT/KR2023/020406**

(87) International publication number:
**WO 2025/127180 (19.06.2025 Gazette 2025/25)**

(54) **OPERATION METHOD OF A SYSTEM FOR CORRECTING POSTURE BY USING ARTIFICIAL
INTELLIGENCE**

BETRIEBSVERFAHREN EINES SYSTEMS ZUR KORREKTUR DER HALTUNG UNTER
VERWENDUNG VON KÜNSTLICHER INTELLIGENZ

PROCÉDÉ DE FONCTIONNEMENT D'UN SYSTÈME DE CORRECTION DE POSTURE À L'AIDE
D'UNE INTELLIGENCE ARTIFICIELLE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**30.07.2025 Bulletin 2025/31**

(73) Proprietor: **POSTUREAI INC.**
**Gyeonggi-do 13488 (KR)**

(72) Inventor: **LEE, Eun Young**
**Seongnam-si**
**Gyeonggi-do 13530 (KR)**

(74) Representative: **Frenkel, Matthias Alexander**
**Wuesthoff & Wuesthoff**
**Patentanwälte und Rechtsanwalt PartG mbB**
**Schweigerstraße 2**
**81541 München (DE)**

(56) References cited:
CN-A- 116 310 083     KR-A- 20210 064 999
KR-A- 20210 064 999     KR-A- 20220 146 290
KR-A- 20230 008 279     KR-B1- 102 593 121
KR-B1- 102 593 121

- **BAZAREVSKY VALENTIN, GRISHCHENKO IVAN,
RAVEENDRAN KARTHIK, ZHU TYLER, ZHANG
FAN, GRUNDMANN MATTHIAS: "BlazePose: On-
device Real-time Body Pose tracking",
ARXIV.ORG, CORNELL UNIVERSITY LIBRARY,
ARXIV.ORG, 1 June 2020 (2020-06-01),
XP093314916, Retrieved from the Internet
<URL:https://arxiv.org/pdf/2006.10204>**

**Description**

[Technical Field]

The present disclosure relates to an operating method of a system for correcting posture using artificial intelligence (AI).

[Background Art]

Musculoskeletal disorders, which are common among modern people, are often caused by incorrect posture or prolonged use of the same posture due to increasingly sedentary lifestyles, computerized work environments, and increasing use of mobile devices, such as smartphones and tablets, and are on the rise. In particular, with sedentary lifestyles, modern people often find themselves in repetitive postures that involve hanging their heads or bending forward at the waist. When this posture continues, the head gradually moves forward from the center of the body, the shoulders slump, and the back curves. Also, the weight of the head causes the neck muscles to strain and the muscles connected to the neck muscles to stiffen, and muscles in the shoulders are always tense to support the weight of the head, making it easier to get tired. In addition, when a person has a curved back, the joints in the spine are directed backward from the center of the body, making it easier for the center of the body to shift or tilt to the left or right.

Since it is very important for modern people to stay in a good posture, chairs, desks, and the like for sedentary lifestyles are ergonomically designed, and technologies are emerging to correct posture.

However, users have not been able to receive appropriate feedback for posture correction until now, and posture correction personalized to each individual user is not appropriately made.

[Related Art Document]

[Patent Document]

(Patent Document 0001) Korean Patent Publication No. 10-1480026 (Dec 31, 2014)

KR20210064999A disclose an operating method of a posture correction system for correcting a posture using artificial intelligence, wherein the posture correction system includes at least one of a server (160), a user terminal (implicit), and a posture sensor (120; 150), the operating method comprising: setting, by the user terminal, reference range information of a good posture on the basis of an input of a user; acquiring daily posture information of the user on the basis of a daily posture sensor (120) which is included in a posture sensor and measures a tilt of the user's spine; outputting, by the user terminal, a message recommending straightening up a posture when the daily posture information is not included in the reference range information; acquiring posture image information of the user on the basis of an exercise posture sensor (150) which is included in the posture sensor and images the user's entire body; acquiring, by the server, three-dimensional posture information by applying the posture image information to a posture estimation machine learning model; determining, by the server, posture similarity between the 3D posture information and predetermined reference posture information; and outputting, by the user terminal, a message recommending correcting the posture when the posture similarity is less than predetermined threshold similarity.

CN116310083A discloses a method for posture assessment using image data.

KR102593121B1 describes a user interface for posture correction systems.

[Disclosure]

[Technical Solution]

The invention is defined by the appended claims.

[Advantageous Effects]

The posture correction system of the present disclosure can acquire three-dimensional (3D) posture information of a user using only one camera. In other words, it is possible to acquire a user's 3D posture information inexpensively and accurately. The posture correction system of the present disclosure acquires 3D posture information and thus can inform a user about a method of 3D posture correction method. Accordingly, it is possible to efficiently and accurately improve the user's posture.

In addition, the posture correction system of the present disclosure can measure a user's posture in conjunction with various posture sensors as well as a camera. Since it is unnecessary to image a user and the user's posture is measured in real time, the user's daily posture can be improved.

[Description of Drawings]

FIG. 1 is a diagram of a user terminal according to an embodiment of the present disclosure.
FIG. 2 is a diagram illustrating a configuration of a posture correction system according to an embodiment of the present disclosure.
FIG. 3 is a flowchart illustrating operations of the posture correction system according to an embodiment of the present disclosure.
FIG. 4 shows values related to automatic range setting and manual range setting according to an embodiment of the present disclosure.
FIG. 5 is a flowchart illustrating operations of the posture correction system according to an embodiment of the present disclosure.
FIG. 6 shows a user interface displayed in the user terminal in relation to range setting according to an embodiment of the present disclosure.
FIG. 7 is a flowchart illustrating operations of the posture correction system according to an embodiment of the present disclosure.
FIG. 8 is a diagram illustrating a posture estimation machine learning model according to an embodiment of the present disclosure.
FIG. 9 is a diagram illustrating an operation of the posture correction system according to an embodiment of the present disclosure.
FIG. 10 is a flowchart illustrating operations of the posture correction system according to an embodiment of the present disclosure.
FIG. 11 shows a screen of the user terminal according to an embodiment of the present disclosure.
FIG. 12 is a diagram illustrating a daily posture object according to an embodiment of the present disclosure.
FIG. 13 is a diagram illustrating an operation of the user terminal according to an embodiment of the present disclosure.
FIG. 14 is a diagram illustrating an operation of the user terminal according to an embodiment of the present disclosure.
FIG. 15 is a diagram illustrating an operation of the user terminal according to an embodiment of the present disclosure.
FIG. 16 is a flowchart illustrating operations of the posture correction system according to an embodiment of the present disclosure.
FIG. 17 shows a screen of the user terminal according to an embodiment of the present disclosure.
FIG. 18 is a diagram illustrating a daily posture object according to an embodiment of the present disclosure.
FIG. 19 is a diagram illustrating an operation of the posture correction system according to an embodiment of the present disclosure.
FIG. 20 is a diagram illustrating an operation of the user terminal according to an embodiment of the present disclosure.

[Modes of the Invention]

Advantages and features of disclosed embodiments and methods of achieving them will become apparent with reference to the embodiments described in detail below in conjunction with the accompanying drawings. However, the present disclosure is not limited to the embodiments disclosed below but will be implemented in various different forms, and the present embodiments are only provided to make the present disclosure complete and fully convey the scope of the invention to those of ordinary skill in the art to which the present disclosure pertains.

Terms used in this specification will be briefly described, and then the disclosed embodiments will be described in detail.

Although terms used herein are selected from among general terms that are currently widely used in consideration of functions in the present disclosure, the terms may be changed in accordance with intentions of those skilled in the art, precedents, the advent of new technology, or the like. When a term is arbitrarily defined by the applicant, the meaning of the term will be described in detail in the corresponding description of the invention. Accordingly, the terms used in the present disclosure should be defined on the basis of the meanings of the terms and the whole context throughout the present disclosure rather than the simple names of the terms.

Singular expressions used herein include plural expressions unless the context clearly indicates otherwise. Also, plural expressions include singular expressions unless the context clearly indicates otherwise.

Throughout the specification, when a part is referred to as "including" any component, the part may further include other components rather than excluding other components unless otherwise designated.

In addition, the term "unit" used herein indicates a hardware or software component, and a "unit" performs certain roles. However, the term "unit" is not limited to software or hardware. A "unit" may be configured to be present in an addressable storage medium or configured to operate one or more processors. Accordingly, as an example, a "unit" includes components, such as software components, object-oriented software components, class components, and task components, processes, functions, attributes, procedures, subroutines, segments of program code, drivers, firmware, microcode, circuitry, data, databases, data structures, tables, arrays, and variables. Functionality provided in components and "units" may be combined into fewer components and "units" or may be further subdivided into additional components and "units."

According to an embodiment of the present disclosure, a "unit" may be implemented using a processor and a memory. The term "processor" should be widely construed as including a general-purpose processor, a central processing unit (CPU), a microprocessor, a digital signal processor (DSP), a controller, a microcontroller, a state machine, and the like. In some environments, a "processor" may indicate an application-specific integrated circuit (ASIC), a programmable logic device (PLD), a field programmable gate array (FPGA), or the like. The term "processor" may indicate a combination of processing devices, such as a combination of a DSP and a microprocessor, a combination of a plurality of microprocessors, a combination of one or more microprocessors combined with a DSP core, or a combination of any other such elements.

The term "memory" should be widely construed as including any electronic component capable of storing electronic information. The term "memory" may indicate various types of processor-readable media such as a random access memory (RAM), a read-only memory (ROM), a non-volatile RAM (NVRAM), a programmable ROM (PROM), an erasable PROM (EPROM), an electrically erasable PROM (EEPROM), a flash memory, a magnetic or optical data storage device, registers, and the like. When a processor can read and/or record information from and/or on a memory, the memory is referred to as "in electronic communication with the processor." A memory integrated into a processor is in electronic communication with the processor.

Hereinafter, embodiments of the present disclosure will be described in detail with reference to the accompanying drawings such that those with ordinary skill in the art to which the present disclosure pertains can easily implement the present disclosure. To clearly describe the present disclosure, parts irrelevant to the description will be omitted in the drawings.

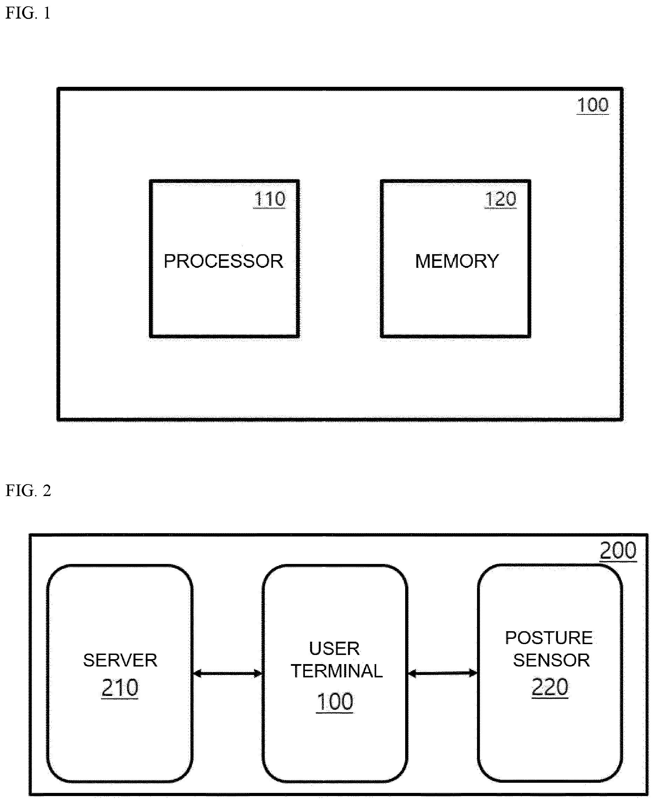

FIG. 1 is a diagram of a user terminal according to an embodiment of the present disclosure.

A user terminal 100 may include a processor 110 and a memory 120. The processor 110 may perform instructions stored in the memory 120. Like the user terminal 100, at least one of a server 210 and a posture sensor 220 may include a processor and a memory. At least some operations performed by the user terminal 100 to be described below may be performed by at least one of the server 210 and the posture sensor 220. On the other hand, at least some operations performed by the server 210 and the posture sensor 220 may be performed by the user terminal 100.

FIG. 2 is a diagram illustrating a configuration of a posture correction system according to an embodiment of the present disclosure.

A posture correction system 200 may include at least one of the server 210, the user terminal 100, and the posture sensor 220. The posture correction system 200 may correct posture using artificial intelligence (AI). More specifically, the posture correction system 200 may accurately measure a user's posture using the AI. Also, the posture correction system 200 may suggest a good posture on the basis of the accurately measured posture of the user. Further, the posture correction system 200 may predict a disease that may develop if the user continually adopts a current posture.

The user terminal 100 is a device used by the user. The user terminal 100 may be at least one of a personal computer (PC), a smartphone, a tablet, and a smart watch. The user terminal 100 may be a device that may be carried by the user. However, the user terminal 100 is not limited thereto. The user terminal 100 may include at least one of an input unit, an output unit, a communication unit, and a control unit. The input unit may be an element for the user to input information. The input unit may include at least one of a keyboard, a mouse, and a touchscreen. The output unit may be an element for outputting information using sound or light. The output unit may include at least one of a touchscreen, a monitor, and a speaker. The control unit may be an element for controlling elements of the user terminal 100. The communication unit may be an element for the user terminal 100 to communicate with at least one of the server 210 and the posture sensor 220. In the present disclosure, the posture sensor 220 may communicate with the user terminal 100, and the user terminal 100 may communicate with the server 210. The server 210 and the posture sensor 220 may exchange information through the user terminal 100. However, the present disclosure is not limited thereto, and the server 210 may directly communicate with the posture sensor 220.

The server 210 may be a device remotely located from the user. The server 210 may be a device fixed at a remote site. In other words, the server 210 may not be a device that may be carried by the user. The server 210 may store information received from a plurality of user terminals. For example, the server 210 may store at least one of daily posture information and three-dimensional (3D) posture information. The server 210 may process information received from the user terminal 100 and transmit result information to the user terminal 100. In the present disclosure, at least one of operations performed by the server 210 may be performed by the user terminal 100. Also, in the present disclosure, at least one of operations performed by the user terminal 100 may be performed by the server 210.

The posture sensor 220 may be a device for measuring a user's posture. The posture sensor 220 may acquire posture information. The posture sensor 220 may include at least one of a daily posture sensor and an exercise posture sensor. The posture information may include at least one of daily posture information and 3D posture information. The daily posture sensor may acquire the daily posture information, and the exercise posture sensor may acquire the 3D posture information.

According to an embodiment of the present disclosure, the daily posture sensor may acquire daily posture information by measuring the user's tilt. The daily posture information may include directional information and magnitude information of the user's tilt. The directional information may indicate a direction in which the user tilts. For example, the directional information may indicate a direction in which the user's spine tilts with respect to the ground. Also, the magnitude information may indicate a magnitude of the user's tilt. For example, the magnitude information may indicate an angle at which the user's spine tilts with respect to the ground. The user's tilt, which is daily posture information, may be at least one of a tilt of the user's upper body and a tilt of the user's spine. In the present disclosure, a tilt of the user's upper body may indicate a tilt of the user's spine. The user's tilt may be at least one of a tilt of the user's spine with respect to the lower body (a longitudinal direction of the thighs) and a tilt of the user's spine with respect to the ground. The directional information of a tilt included in the daily posture information may include forward, backward, left, and right directions. The directional information included in the daily posture information may include at least one of forward, backward, left, right, forward left, backward left, forward right, and backward right directions. However, the directional information is not limited thereto and may indicate a direction in which the user's spine tilts using one value among 0 degrees to less than 360 degrees. The magnitude information included in the daily posture information may indicate an angle between an axis perpendicular to the ground and the spine. In other words, the magnitude information included in the daily posture information may have a smaller value when the user's spine is more perpendicular to the ground, and may have a larger value when the user's spine is more parallel to the ground. Also, the magnitude information included in the daily posture information may have a smaller value when an angle of the user's spine with respect to the thighs is closer to 180 degrees, and may have a large value when the angle becomes smaller than 180 degrees.

According to an embodiment of the present disclosure, the exercise posture sensor may acquire posture image information. At least one of the user terminal 100 and the server 210 may acquire 3D posture information indicating the user's skeletal posture on the basis of the posture image information. The exercise posture sensor may include at least one of a camera, a depth camera, and an infrared sensor.

The 3D posture information based on the exercise posture sensor may be information including an angle of at least one joint included in the user's body. For example, the 3D posture information may include at least one of an angle of an elbow, an angle of a knee, an angle of the waist, an angle of the neck, an angle of an arm with respect to the torso, an angle of a thigh bone with respect to the torso, an angle of a wrist, and an angle of an ankle. However, the 3D posture information is not limited thereto and may indicate a set of coordinates of the user's body feature nodes. One body feature node may correspond to one of the head, the neck, the left shoulder, the right shoulder, an elbow, a hand, the pelvis, a knee, and a foot. A set of a plurality of physical feature nodes included in the user may be the 3D posture information. The body feature nodes may include identification information and coordinate values. For example, the identification information of the body feature nodes may indicate one of the head, the neck, the left shoulder, the right shoulder, an elbow, a hand, the pelvis, a knee, and a foot. Connections of a plurality of body feature nodes may be determined on the basis of the identification information of the body feature nodes. For example, the head may be connected to the neck, the shoulders may be connected to the elbows, and the elbows may be connected to the hands. The coordinate values of the body feature nodes may indicate 3D coordinate values of the body feature nodes in a virtual space. While the posture image information of the exercise posture sensor indicates the user's two-dimensional (2D) posture, the 3D posture information obtained by adding depth information to the 2D information may indicate the user's posture in a 3D space.

The posture sensor 220 may be, but is not limited to, a device independent of the user terminal 100 and included in the user terminal 100. The posture sensor 220 may be included in a wearable device. For example, the posture sensor 220 may be included in a smart seat. The smart seat may include pressure sensors for measuring which part of the seat is more pressurized when the user sits on the seat. When a pressure value is measured at a pressure sensor on a front side, the user's posture may be considered to be tilting forward.

The posture sensor 220 according to the present disclosure may include a sensor for sensing motions or postures of users' bodies in a 3D space. The posture sensor 220 may include a 3-axis gyro sensor for measuring 360-degree directions. The posture sensor 220 may include a 3-axis acceleration sensor, the 3-axis gyro sensor, or a 3-axis geomagnetism sensor. The posture sensor 220 may include an inertial measurement unit (IMU). Using the IMU, the posture sensor 220 may measure the user's posture angle, posture angular velocity, posture angle acceleration, acceleration, and the like. The user's posture angle, posture angular velocity, posture angle acceleration, acceleration, or the like may be included in one of the user's posture information and daily posture information.

According to an embodiment of the present disclosure, the posture sensor 220 may measure forward, backward, left, and right directions using the 3-axis gyro sensor, the 3-axis acceleration sensor, the 3-axis geomagnetism sensor, or the like and measure the user's motion in 360-degree directions, that is, daily body posture information. Since various types of sensors may be used to measure the user's posture area in forward, backward, left, right, and 360-degree directions, the posture sensor 220 is not particularly limited to any one form. According to an embodiment of the present disclosure, only when sensing data acquired by the posture sensor 220 is not limited to posture information of a forward or backward tilt but includes posture information of left, right, and 360-degree directions, is it possible to recommend an appropriate posture correction exercise. Accordingly, posture data related to 360-degree directions may be necessary for an exercise recommendation service according to an embodiment of the present disclosure.

Operations of the posture correction system 200 will be described below.

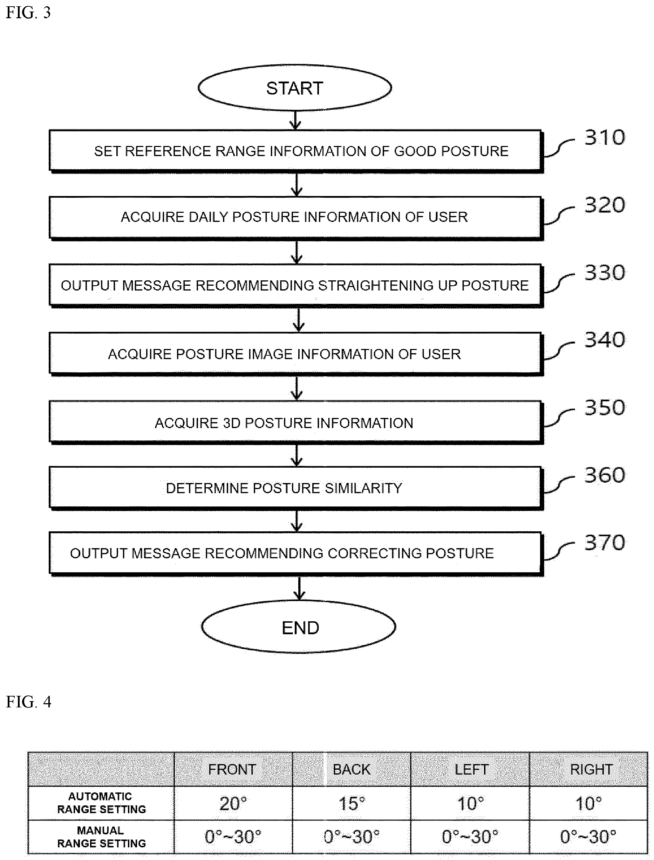

FIG. 3 is a flowchart illustrating operations of the posture correction system according to an embodiment of the present disclosure.

The user terminal 100 may perform an operation 310 of setting reference range information of a good posture on the basis of a user's input. The reference range information may be information for determining whether the user's daily posture information corresponds to a good posture or a bad posture. The reference range information may be determined to be 0 degrees or more and x degrees or less. x may be a predetermined value or a value set by the user. When the user's spine is perpendicular to the ground, a tilt of the spine may be 0 degrees. Also, a posture with the spine perpendicular to the ground may be a good posture.

When the user's daily posture information is included in the reference range information, the posture correction system 200 may determine the user's posture to be a good posture. When the user's daily posture information is not included in the reference range information, the posture correction system 200 may determine the user's posture to be a bad posture.

The operation 310 of setting the reference range information will be described with reference to FIGS. 4 to 6.

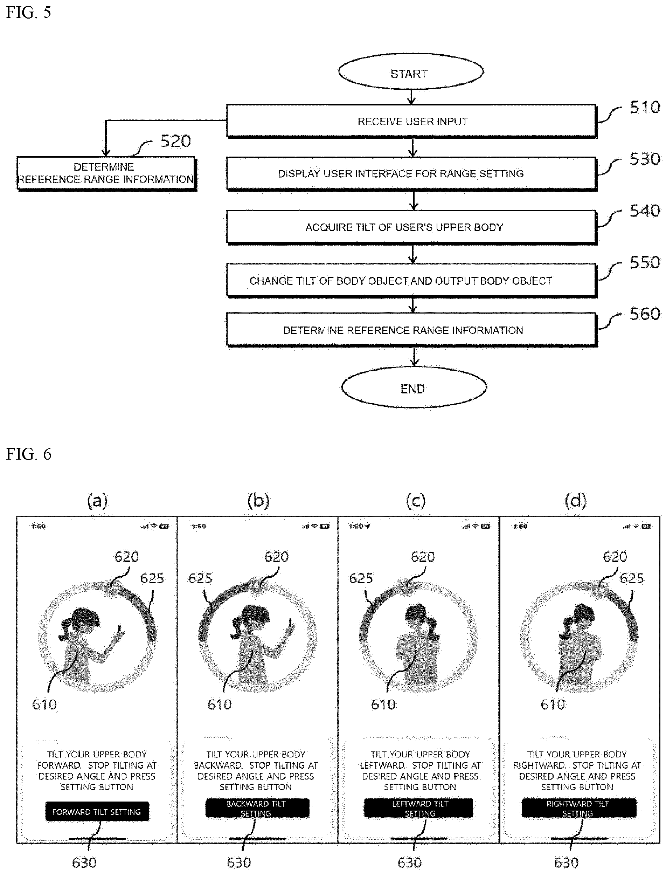

FIG. 4 shows values related to automatic range setting and manual range setting according to an embodiment of the present disclosure. FIG. 5 is a flowchart illustrating operations of the posture correction system according to an embodiment of the present disclosure. FIG. 6 shows a user interface displayed in the user terminal in relation to range setting according to an embodiment of the present disclosure.

The operation 310 of setting the reference range information may include the following operations.

Referring to FIGS. 4 and 5, the user terminal 100 may perform an operation 510 of receiving a user input related to reference range information from the user. When a program related to the posture correction system is installed on the user terminal 100 for the first time, the user terminal 100 may display a user interface for setting reference range information. The user may input a user input indicating whether to automatically or manually set reference range information to the user terminal 100.

When the user input indicates automatic range setting, the user terminal 100 may perform an operation 520 of determining predetermined initial range information as the reference range information. The initial range information may be information prestored in the user terminal 100. The initial range information may be set for the user's forward, backward, left, and right directions. However, the initial range information is not limited thereto and may be set for at least one of the user's forward, backward, left, right, forward left, backward left, forward right, and backward right directions.

For example, referring to FIG. 4, the initial range information may be 20 degrees for the forward direction, 15 degrees for the backward direction, 10 degrees for the left direction, and 10 degrees for the right direction. The posture correction system 200 may determine the initial range information as the reference range information. The reference range information being 20 degrees for the forward direction may represent that a forward tilt of 0 degrees to 20 degrees is a good posture. 0 degrees represents that the user's spine is perpendicular to the ground and is naturally included in a good posture range. Accordingly, the reference range information may be indicated as only a maximum of the tilt. Likewise, the reference range information being 10 degrees for the left direction may represent that a left tilt of 0 degrees to 10 degrees is a good posture.

The posture correction system 200 may determine whether the user's posture is good on the basis of the reference range information. For example, when the user bends forward 20 degrees or less, the posture correction system 200 may determine the user's posture to be good. On the other hand, the user bends forward more than 20 degrees, the posture correction system 200 may determine the user's posture to be bad. When the reference range information is automatically determined as described above, user inputs are minimized, and thus the user's convenience can be improved.

When the user input indicates manual range setting, the user terminal 100 may perform an operation 530 of displaying a user interface for range setting. The user interface for range setting may be a screen for setting reference range information. Referring to FIG. 4, the user terminal may set reference range information to a partial range of 0 degrees to 30 degrees on the basis of the user interface for range setting. More specifically, reference range information for the forward direction may be set to a partial range between 0 degrees and 30 degrees, reference range information for the backward direction may be set to a partial range between 0 degrees and 30 degrees, reference range information for the left direction may be set to a partial range between 0 degrees and 30 degrees, and reference range information for the right direction may be set to a partial range between 0 degrees and 30 degrees. As described above, 0 degrees is naturally included in a good posture range, and thus the reference range information may be indicated as only a maximum of the tilt determined to be a good posture. In other words, reference range information for the forward direction may be set to one value of 0 degrees to 30 degrees, reference range information for the backward direction may be set to one value of 0 degrees to 30 degrees, reference range information for the left direction may be set to one value of 0 degrees to 30 degrees, and reference range information for the right direction may be set to one value of 0 degrees to 30 degrees.

The user interface for range setting according to an embodiment of the present disclosure may be as shown in FIG. 6. For example, referring to FIG. 6A, the user terminal 100 may display a screen for acquiring reference range information for the forward direction. Also, referring to FIG. 6B, the user terminal 100 may display a screen for acquiring reference range information for the backward direction. Also, referring to FIG. 6C, the user terminal 100 may display a screen for acquiring reference range information for the left direction. Also, referring to FIG. 6D, the user terminal 100 may display a screen for acquiring reference range information for the right direction.

When the user interface for range setting is displayed as shown in FIG. 6, the user may set reference range information on the basis of guidance from the user terminal 100. More specifically, the user terminal 100 may perform an operation 540 of acquiring a tilt of the user's spine on the basis of the daily posture sensor. The user terminal 100 may display a message to the user that recommends tilting the user's spine at an angle to be set. The user equipped with the daily posture sensor may tilt his or her spine to an angle which is considered a normal tilt. The daily posture sensor may measure the tilt of the user's spine. The tilt of the spine may indicate at least one of a tilt of the spine with respect to the ground and a tilt of the spine with respect to a leg. The user terminal 100 may acquire the tilt of the spine in each of the forward, backward, left, and right directions.

In the operation 540, it has been described that the tilt of the spine is acquired using the daily posture sensor, but the present disclosure is not limited thereto. In the operation 540, the user terminal 100 may acquire the tilt of the spine using the exercise posture sensor.

Referring to FIG. 5, the user terminal 100 may perform an operation 550 of changing a tilt of a body object 610 representing at least a part of a human body included in the user interface on the basis of the tilt of the spine and outputting the body object 610. The body object 610 may be an object representing the user's spine. However, the body object 610 is not limited thereto and may represent the user's entire body. The body object 610 may be an illustration corresponding to the user's body. However, the body object 610 is not limited thereto and may be an image of the user captured using a camera. The user terminal 100 may control a tilt of the body object 610 on the basis of the tilt of the spine acquired in the operation 540. Accordingly, the user can check the tilt of his or her body with his or her eyes. The user can determine whether the tilt of his or her body is normal. The user terminal 100 may induce the user to stay in a good posture by providing an interface for accurately determining the reference range information.

Referring to FIG. 6, the user terminal 100 may output a value 620 of the tilt of the user's spine. The user terminal 100 may notify the user which value the reference range information will be set to by outputting the value 620 of the tilt of the spine.

The user terminal 100 may include an object 625 representing a predetermined limit tilt. The object 625 representing the limit tilt may have a curved shape. For example, the limit tilt may be 30 degrees. The limit tilt may be a maximum of the reference range information. The reference range information may be determined to be a limit tilt value or less.

Referring to FIG. 5, the user may maintain the tilt of the spine to be set by himself or herself. Here, when the user checks at least one of the tilt of the body object 610 and the value 620 and inputs an input for an object 630 representing confirmation, the user terminal 100 may perform an operation 560 of determining the reference range information to be 0 degrees to the tilt of the spine. The object 630 representing confirmation may be a button shown on the display. However, the object 630 is not limited thereto and may be implemented as a physical button. When the input for the object 630 is received, the user terminal 100 may determine the reference range information to be 0 degrees to the tilt of the spine. The tilt of the spine may be a tilt of the spine maintained by the user.

As described above, the user tries a posture to determine the reference range information which is the range of a good posture, and thus the posture correction system of the present disclosure does not require the user to unnecessarily stay in an uncomfortable posture to correct the posture. Also, since the user tries a posture to set the reference range information, there is less trial and error, and the user terminal 100 can rapidly determine the reference range information. A good posture can be different for everyone. The posture correction system 200 of the present disclosure can recommend a good posture which is different for everyone to the user.

Referring back to FIG. 3, the user terminal 100 may perform an operation 320 of acquiring the user's daily posture information on the basis of the daily posture sensor that is included in the posture sensor and measures a tilt of the user's tilt.

The daily posture sensor may acquire daily posture information by measuring the user's tilt. The daily posture sensor may include at least one of a pressure sensor and an inertial sensor for measuring the user's tilt. The daily posture sensor may be included in a wearable device of the user. The daily posture sensor may be disposed close to the user's chest or back. The daily posture sensor may be included in a chair or a smart seat. The daily posture sensor may measure the daily posture information which is the user's tilt. The user's tilt may be a tilt of the user with respect to the ground or a tilt of the user's spine with respect to the lower body. Also, the user's tilt may be a tilt of the user's neck bone with respect to the ground or a tilt of the user's neck bone with respect to the spine. Although the daily posture information measured by the daily posture sensor is referred to as a "tilt of the spine" for convenience of description, the daily posture information is not limited thereto.

When the daily posture information is not included in the reference range information, the user terminal 100 may perform an operation 330 of outputting a message that recommends straightening up the posture. For example, when the reference range information for the forward direction is 0 degrees or more and 15 degrees or less and the daily posture information is 25 degrees, the daily posture information is not included in the reference range information. In this case, the user terminal 100 may determine the user's posture as a bad posture. Also, the user terminal 100 may output a message that recommends straightening up the posture. The user may correct his or her posture on the basis of the message. Whenever the user is not in a good posture, the posture correction system 200 of the present disclosure may output the message to induce the user to stay in a good posture. Accordingly, the user's posture may be gradually corrected.

Also, the posture correction system 200 of the present disclosure acquires a relatively simple tilt of the user as daily posture information, and thus it is unnecessary to prepare a complex and expensive sensor. In addition, since the posture correction system 200 of the present disclosure simply compares values and recommends a good posture, no high computing capability is necessary. Accordingly, a message for correcting a posture can be rapidly output. In other words, since there is no delay, the user can immediately recognize that he or she is in a bad posture and correct his or her posture.

A process in which the posture correction system 200 acquires daily posture information using the daily posture sensor and induces the user to stay in a good posture has been described above. When the posture correction system 200 utilizes daily posture information acquired by the daily posture sensor, the user can rapidly have feedback, but it is difficult to know the user's accurate posture. In particular, when the posture correction system 200 recommends an exercise for correcting the user's posture, it is difficult to determine whether the user accurately follows the recommended exercise. The exercise recommended by the posture correction system 200 is to correct the user's posture, and when the user does not have an accurate posture indicated by the exercise, the user's posture may actually worsen. For this reason, the posture correction system may further employ the exercise posture sensor.

First, a process in which the posture correction system 200 recommends an exercise to the user through the user terminal 100 will be described. This process may be performed between the operations 330 and 340.

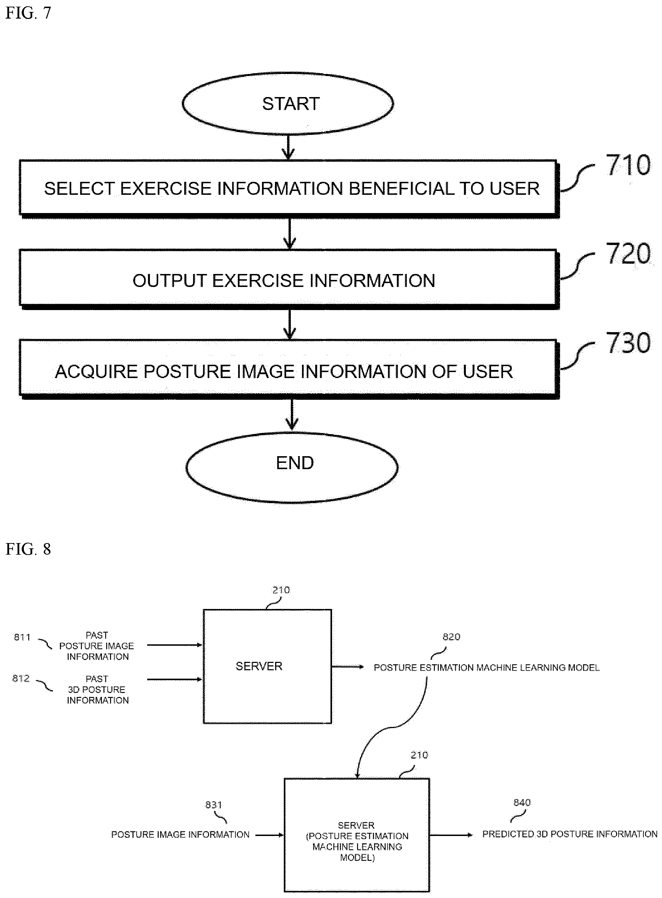

FIG. 7 is a flowchart illustrating operations of the posture correction system according to an embodiment of the present disclosure.

The user terminal 100 may perform an operation 710 of selecting exercise information beneficial to the user from among a plurality of pieces of candidate exercise information on the basis of the daily posture information. The user terminal 100 may acquire cumulative daily posture information by accumulating the daily posture information. The cumulative daily posture information may be information obtained by accumulating the daily posture information during a predetermined collection time. The predetermined collection time may be one of one day, one week, and one month.

The plurality of pieces of candidate exercise information may be identification information of exercises that are recommendable to the user. The plurality of pieces of candidate exercise information may be about exercises for improving the user's posture. At least one of the plurality of pieces of candidate exercise information may be recommended to the user in accordance with a type of posture that the user is typically in.

The user terminal 100 may acquire information about which posture the user is typically in on the basis of the cumulative daily posture information. For example, the user terminal 100 may acquire information about which direction among the forward, backward, left, and right directions the user tilts in for a long time. The user terminal 100 may store an exercise information selection table. The exercise information selection table may be used for selecting exercise information. The exercise information selection table may be a table in which exercise information corresponds to a direction of a posture that the user is in for the longest time. For example, when the user tilts forward for the longest time, the user terminal 100 may select exercise information corresponding to a case of tilting forward for the longest time from the exercise information selection table.

According to various embodiments of the present disclosure, the user terminal 100 may acquire sorted tilt directions by sorting times for which the user tilts forward, backward, leftward, and rightward in order of time length. For example, the user may tilt forward for the longest time, tilt leftward for the second longest time, tilt rightward for the third longest time, and tilt backward for the least time. In this case, the user terminal 100 may acquire the forward, left, right, and backward directions as sorted tilt directions. The user terminal 100 may store the exercise information selection table in which the sorted tilt directions correspond to exercise information. The user terminal 100 may select exercise information corresponding to a sorted tilt direction from the exercise information selection table.

According to various embodiments of the present disclosure, the user terminal 100 may acquire bad posture times by direction on the basis of the cumulative daily posture information. The user terminal 100 may select exercise information on the basis of a direction corresponding to the longest bad posture time. More specifically, the user terminal 100 may select exercise information corresponding to the longest bad posture time from the exercise information selection table. For example, when the forward direction corresponds to the longest bad posture time, the user terminal 100 may select exercise information corresponding to the forward direction. Bad posture times will be described in detail below.

According to various embodiments of the present disclosure, the user terminal 100 may acquire sorted tilt directions by sorting tilt directions in decreasing order of bad posture time. For example, tilt directions sorted in decreasing order of bad posture time may be the forward, left, right, and backward directions. The user terminal 100 may select exercise information corresponding to a sorted tilt direction from the exercise information selection table.

The operation 710 of selecting exercise information may further include the following operations. The user terminal 100 may perform an operation of acquiring disease information from the user. The disease information may include a disease code. The user terminal 100 may select one disease from a predetermined list of a plurality of pieces of disease information on the basis of the user's input.

The user terminal 100 may perform an operation of selecting partial candidate exercise information by excluding exercise information harmful to the user from the plurality of pieces of candidate exercise information on the basis of the disease information. The user terminal 100 may store a harmful exercise table in advance. The harmful exercise table may be a table in which disease information corresponds to harmful exercise information. The user terminal 100 may select harmful exercise information corresponding to the disease information from the harmful exercise table. The harmful exercise information may be information related to an exercise proposing a posture that the user cannot take or an exercise that may exacerbate the user's disease. The user terminal 100 may acquire the partial candidate exercise information by excluding the harmful exercise information from the plurality of pieces of candidate exercise information. The partial candidate exercise information may be a subset of the plurality of pieces of candidate exercise information.

The user terminal 100 may perform an operation of selecting exercise information based on the daily posture information from the partial candidate exercise information. Unlike the operation 710, the user terminal 100 may not select exercise information from among the plurality of pieces of candidate exercise information but may select exercise information from the partial candidate exercise information. As described above, the user terminal 100 does not recommend harmful exercise information to the user such that the posture correction system 200 does not exacerbate the user's disease. Also, the posture correction system 200 does not provide the user with exercise information including a posture that the user cannot take and thus can only provide the user with exercises that are efficient for improving the user's posture.

The user terminal 100 may perform an operation 720 of outputting the exercise information. The user terminal 100 may output identification information of the exercise information. Also, the user terminal 100 may output a document that describes a posture to be taken by the user, still images that show postures to be taken by the user in chronological order, or a video included in the exercise information. The user may take a posture on the basis of the still images or the video output by the user terminal 100. When the user takes the posture corresponding to the exercise information, the user's posture can be improved.

When the user takes a posture on the basis of the exercise information, the user terminal 100 may perform an operation 730 of acquiring posture image information of the user on the basis of the exercise posture sensor. The operation 730 of acquiring posture image information may correspond to the operation 340.

The process of FIG. 7 may be performed between the operations 330 and 340. However, operations performed between the operations 330 and 340 are not limited to FIG. 7, and other operations may be performed. Also, the operations of FIG. 7 may not be performed between the operations 330 and 340. The operation 340 will be described with reference to FIG. 3 again.

The server 210 or the user terminal 100 may perform the operation 340 of acquiring the posture image information of the user on the basis of the exercise posture sensor that is included in the posture sensor and images the user's entire body. The exercise posture sensor may include at least one of a camera, a depth camera, and an infrared sensor. The posture image information may be a 2D image having color information as pixel values. The color information may be values based on wavelengths of visible light. However, the posture image information is not limited thereto and may be an image having depth values as pixel values. A depth value may indicate the distance from the exercise posture sensor to an object to be imaged.

The server 210 may perform an operation 350 of acquiring 3D posture information by applying at least one of the posture image information and the daily posture information to a posture estimation machine learning model. The posture estimation machine learning model may be a model for outputting 3D posture information on the basis of at least one of posture image information and daily posture information.

A posture estimation machine learning model 820 will be described with reference to FIG. 8.

FIG. 8 is a diagram illustrating a posture estimation machine learning model according to an embodiment of the present disclosure.

A posture estimation machine learning model may be a model generated in accordance with "DRPose3D: Depth Ranking in 3D human Pose Estimation." The posture estimation machine learning model 820 may be a machine learning model for predicting 3D posture information from posture image information. The posture estimation machine learning model 820 may be implemented on the basis of a machine learning model such as a convolutional neural network (CNN). The posture estimation machine learning model 820 may utilize past posture image information 811 and past 3D posture information 812 as training data. The past posture image information 811 may be a target of analysis, and the past 3D posture information 812 may be label information acquired by a person or device. The past 3D posture information 812 may be a ground truth value. The past posture image information 811 and the past 3D posture information 812 may correspond to each other on a one-to-one basis. Posture information of a person shown in a 3D space which is included in the past posture image information 811 may be the past 3D posture information 812.

The server 210 may update the posture estimation machine learning model 820 using forward propagation and backpropagation. The server 210 may perform machine learning on correlation of the past 3D posture information 812 with the past posture image information 811. When the accuracy of the posture estimation machine learning model 820 is sufficiently high, the server 210 may stop updating the posture estimation machine learning model 820. The server 210 may transmit the posture estimation machine learning model 820 to the user terminal 100 or another server. The server 210 may store the posture estimation machine learning model 820.

According to FIG. 8, the server 210 utilizes the past posture image information 811 and the past 3D posture information 812 to generate the posture estimation machine learning model 820. However, the present disclosure is not limited thereto. The server 210 may generate the posture estimation machine learning model 820 on the basis of the past posture image information 811, the past 3D posture information 812, and past daily posture information. The server 210 may perform machine learning on correlation of the past 3D posture information 812 with the past posture image information 811 and the past daily posture information.

The server 210 may receive posture image information 831 from the user terminal 100 or the posture sensor 220. The posture image information 831 may be information acquired by the exercise posture sensor included in the posture sensor 220. A process of acquiring posture image information has been described above in the operation 340. The server 210 may perform an operation 350 of acquiring 3D posture information 840 by applying at least one of the posture image information 831 and the daily posture information to the posture estimation machine learning model 820. The 3D posture information 840 acquired by applying the information to the posture estimation machine learning model 820 may be predicted information. The 3D posture information 840 predicted by the posture estimation machine learning model 820 may be almost the same as actual 3D posture information.

Referring to FIG. 8, the posture estimation machine learning model 820 may only receive the posture image information 831 and determine the predicted 3D posture information 840. However, the present disclosure is not limited thereto. The posture estimation machine learning model 820 may further receive the daily posture information and determine the predicted 3D posture information 840. In this case, the server 210 may utilize the posture estimation machine learning model 820 that performs machine learning on the correlation of the past 3D posture information 812 with the past posture image information 811 and the past daily posture information through machine learning.

The predicted 3D posture information 840 and the past 3D posture information 812 may be included in 3D posture information. While posture image information represents the user's posture in a 2D plane, 3D posture information may include information for expressing the user's posture in a 3D space. In other words, 3D posture information may include depth information.

According to an embodiment of the present disclosure, 3D posture information may include an angle of at least one predetermined joint included in the user's body. 3D posture information may include identification information corresponding to a body feature node. In other words, 3D posture information may include identification information and an angle of a joint corresponding to the identification information. Here, at least some body feature nodes may correspond to joints. Alternatively, body feature nodes may include at least one joint. The at least one predetermined joint may include at least one of the neck, an elbow, a knee, the waist, the pelvis, a wrist, and an ankle. Also, the 3D posture information may include at least one of an angle of an elbow, an angle of a knee, an angle of the waist, an angle of the neck, an angle of an arm with respect to the torso (an angle of the shoulder), an angle of a femur with respect to the torso (an angle of the pelvis), an angle of a wrist, and an angle of an ankle. Relationships between a plurality of joints may be determined in advance. For example, the neck may be connected to the shoulders through skeletal edges corresponding to the collarbones, and the shoulders may be connected to the elbows through skeletal edges corresponding to the humeri. The distances between the plurality of joints may be estimated by the posture estimation machine learning model 820 or determined in advance. When the angles of the plurality of joints are determined, the relationships between the plurality of joints are determined in advance, and the distances between the plurality of joints are determined as described above, the posture correction system may generate a skeletal model representing the overall shape of the user's body. The skeletal model may be represented in a 3D space.

According to various embodiments of the present disclosure, the 3D posture information may include a set of coordinates of the user's body feature nodes. One body feature node may correspond to a point of the body. The point of the body may include at least one of the head, the neck, the left shoulder, the right shoulder, an elbow, a hand, the pelvis, a knee, and a foot. In other words, one body feature node may correspond to one of the head, the neck, the left shoulder, the right shoulder, an elbow, a hand, the pelvis, a knee, and a foot. A set of a plurality of body feature nodes included in the user may be 3D posture information. A body feature node may include identification information and coordinate values. For example, the identification information of the body feature node may represent one of the head, the neck, the left shoulder, the right shoulder, an elbow, a hand, the pelvis, a knee, and a foot. Connections of the plurality of body feature nodes may be determined on the basis of identification information of the body feature nodes. For example, the head may be connected to the neck, the shoulders may be connected to the elbows, and the elbows may be connected to the hands. Coordinate values included in a body feature node may be 3D coordinate values representing a position of the body feature node in an actual space or a virtual space. When the coordinates of the body feature nodes are determined and the connections of the body feature nodes are determined in advance on the basis of the identification information as described above, the posture correction system may generate a skeletal model representing the overall shape of the user's body. At least one of the server 210 and the user terminal 100 may display the skeletal model. The skeletal model may correspond to the 3D posture information.

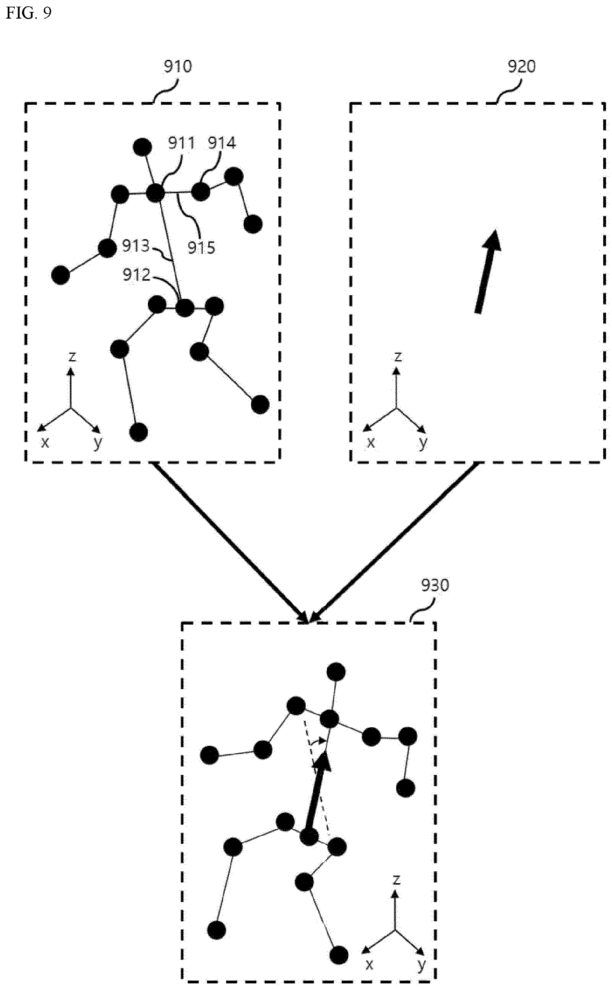

FIG. 9 is a diagram illustrating an operation of the posture correction system according to an embodiment of the present disclosure.

3D posture information may include a plurality of body feature nodes at least partially corresponding to joints constituting a body and skeletal edges connecting the plurality of body feature nodes. Some body feature nodes may correspond to joints, but other body feature nodes may correspond to hands or feet rather than joints. Various body parts may correspond to body feature nodes in accordance with a design of the posture correction system 200.

FIG. 9 shows a skeletal model 910. The posture correction system 200 may generate the skeletal model 910 corresponding to the 3D posture information acquired by the posture estimation machine learning model 820. The skeletal model 910 may be displayed in a 3D space. For example, the 3D posture information may include a first body feature node 911 corresponding to the neck and a second body feature node 912 corresponding to the center of the pelvis. Each of the first body feature node 911 and the second body feature node 912 may include identification information and coordinate information. However, the 3D posture information is not limited thereto. The 3D posture information may also include a first skeletal edge 913 connecting the first body feature node 911 and the second body feature node 912. The first skeletal edge 913 may correspond to the spine.

Further, the 3D posture information may include a third body feature node 914 corresponding to a shoulder. In addition, the 3D posture information may include a second skeletal edge 915 connecting the first body feature node 911 and the third body feature node 914. The second skeletal edge 915 may correspond to the collarbones.

As described above, the user terminal 100 may perform the operation 320 of acquiring daily posture information. Daily posture information 920 acquired in the operation 320 may be displayed as a vector in the 3D space. The vector of the daily posture information 920 may indicate an extension direction of the spine. The vector of the daily posture information 920 may indicate an extension direction of the spine from the pelvis toward the head. However, the vector direction is not limited thereto and may be an extension direction of the spine from the head toward the pelvis. The user terminal 100 acquires daily posture information on the basis of the posture sensor 220 including the inertial sensor and thus can acquire a tilt of the user's spine with respect to the ground. Accordingly, the user terminal 100 may display the daily posture information 920 in the 3D space.

While performing the operation 350 of acquiring 3D posture information, the posture correction system 200 may further perform the following process.

At least one of the server 210 and the user terminal 100 may perform an operation of causing an angle of a skeletal edge, which is included in the 3D posture information and corresponds to the spine, with respect to the ground to correspond to the daily posture information. More specifically, the skeletal edge corresponding to the spine may be the first skeletal edge 913. The server 210 or the user terminal 100 may match the extension direction of the first skeletal edge 913 to a direction of the daily posture information 920. Accordingly, the posture correction system 200 of the present disclosure can accurately determine the tilt of the spine with respect to the ground and accurately determine an angle of the skeletal model with respect to gravity. Therefore, the posture correction system 200 can determine whether the user is accurately in a posture included in the exercise information recommended to the user.

The reason that the posture correction system 200 corrects the 3D posture information on the basis of the daily posture information is as follows. The skeletal model 910 corresponding to the 3D posture information is acquired on the basis of the exercise posture sensor. The exercise posture sensor may not include angle information based on the ground. For example, the exercise posture sensor may be a camera, and the camera captures an object with a horizontal direction thereof generally parallel to the ground, but depending on an environment, the horizontal direction thereof may not be parallel to the ground. In this case, it may be difficult for 3D posture information to include information on a tilt of the spine with respect to the ground. This is because the horizontal direction of posture image information captured by the camera is not parallel to the ground. Further, when the ground is not perpendicular to gravity, even if the camera is kept parallel to the ground and captures posture image information, no information on gravity is included in 3D posture information based on the posture image information.

However, a force applied by the gravity to the spine may be an important factor for determining whether the user is in a good posture. This is because, when the extension direction of the spine is tilted at a large angle with respect to the direction of gravity, a great force may be applied to the spine, and this may be determined to not be a good posture. Accordingly, the 3D posture information based on the exercise posture sensor that does not reflect any information on the direction of gravity may be a serious problem. Therefore, the posture correction system 200 of the present disclosure may correct the 3D posture information on the basis of the daily posture information.

Referring to FIG. 9, the posture correction system may acquire a skeletal model 930 by rotating the skeletal model 910. More specifically, the posture correction system may acquire the skeletal model 930 by rotating the skeletal model 910 such that the direction of the first skeletal edge 913 included in the skeletal model 910 matches the direction of the daily posture information 920. For example, while a spinal tilt of the skeletal model 910 may be included in the reference range information and thus may be a good posture, the spinal tilt of the skeletal model 930 may not be included in the reference range information and thus may be a bad posture. Accordingly, the posture correction system 200 may display a message recommending changing the posture. The user may see the message and correct his or her posture. Therefore, the posture correction system 200 can recommend an exercise for precisely correcting the user's posture and determine whether the user is in a posture based on the recommended exercise.

Referring back to FIG. 3, at least one of the server 210 and the user terminal 100 may perform an operation 360 of determining posture similarity between the 3D posture information and the predetermined reference posture information. The predetermined reference posture information may be information for determining whether the user is accurately taking a posture based on the exercise information. The reference range information may be acquired on the basis of an exercise expert's posture. A case where the server 210 performs the operation 360 will be described below, but the present disclosure is not limited thereto. The user terminal 100 may perform the operation 360.

As described above, the 3D posture information may include identification information corresponding to the body feature nodes and angles of joints corresponding to the identification information. The reference posture information may also include identification information corresponding to body feature nodes and angles of joints corresponding to the identification information. The server 210 may determine posture similarity between joint angles of 3D posture information and reference posture information having the same identification information. For example, the posture similarity may be determined to be a difference ratio in accordance with the following equation.

Difference ratio = |joint angle of 3D posture information-joint angle of reference posture information|/joint angle of reference posture information

When the difference ratio is smaller, the posture similarity may be larger. The above equation is about one joint, and an average of difference ratios of all joints may be used as a difference ratio of all the joints. However, a similarity determination method is not limited thereto, and various similarity determination methods may be used to determine posture similarity.

When the posture similarity is less than predetermined threshold similarity, the user terminal 100 may perform an operation of outputting a message recommending correcting the posture. The user may check the message and correct his or her posture to be similar to a posture provided in the exercise information. Therefore, the user can take an accurate posture, and effects of the exercise for correcting the posture can be maximized accordingly.

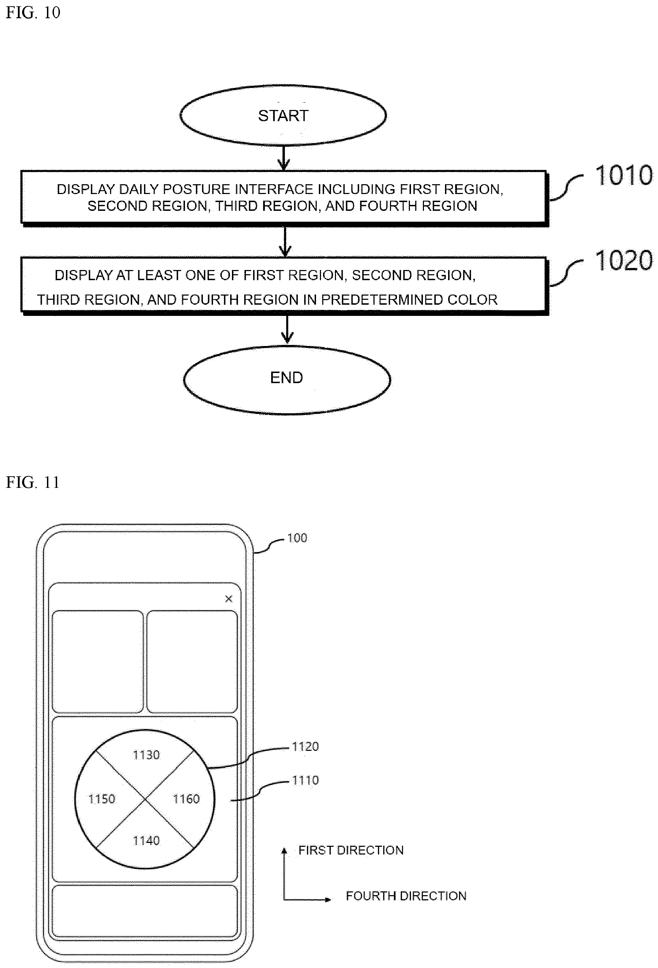

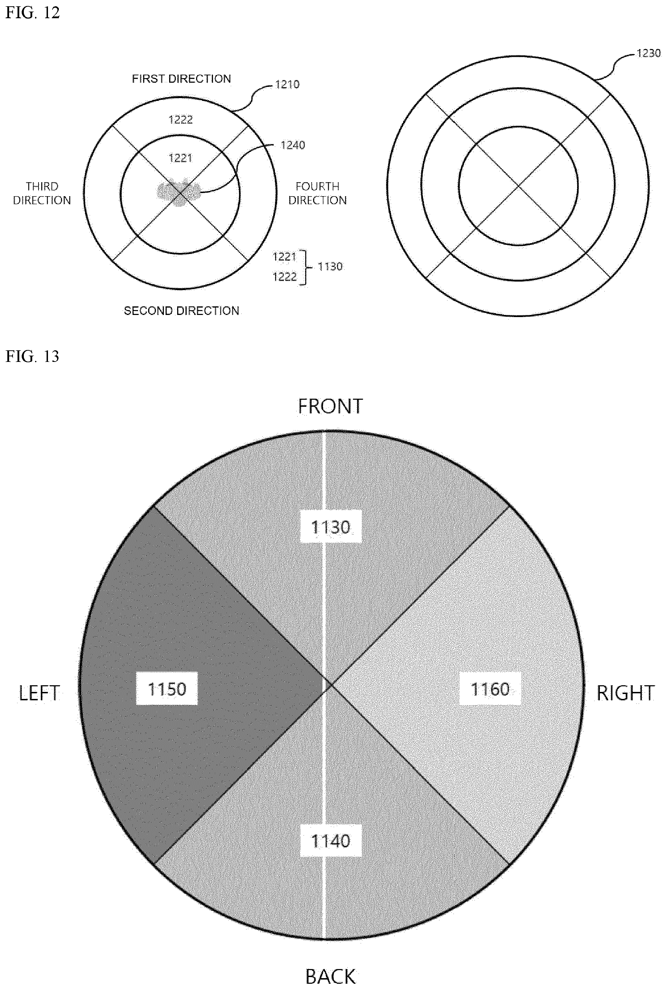

FIG. 10 is a flowchart illustrating operations of the posture correction system according to an embodiment of the present disclosure. FIG. 11 shows a screen of the user terminal according to an embodiment of the present disclosure. FIG. 12 is a diagram illustrating a daily posture object according to an embodiment of the present disclosure.

The operation 320 of acquiring daily posture information may include operations of FIG. 10.

Referring to FIG. 10, the user terminal 100 may perform an operation 1010 of displaying a daily posture object 1120 including a first region, a second region, a third region, and a fourth region. The daily posture object 1120 may show 360 degrees around the user. 360 degrees around the user may be surroundings including front, back, left, and right sides. FIGS. 11 to 15 show some embodiments of a daily posture object, and it is to be noted that a daily posture object can have a different shape from those shown in FIGS. 11 to 15.

In this regard, referring to FIG. 11, the user terminal 100 may include the output unit. The output unit may include a touchscreen. The user terminal 100 may display a daily posture object display region 1110. In the daily posture object display region 1110, the daily posture object 1120 may be displayed. The daily posture object 1120 may be an object showing statistics based on daily posture information. The daily posture object 1120 may include at least one of a first region 1130, a second region 1140, a third region 1150, and a fourth region 1160.

The first region 1130 may be present in a first direction from the daily posture object 1120. The first region 1130 may be a region for representing that the user tilts in the first direction. For example, the first region 1130 may be a region for representing that the user tilts forward.

The second region 1140 may be present in a second direction from the daily posture object 1120. The second region 1140 may be a region for representing that the user tilts in the second direction. For example, the second region 1140 may be a region for representing that the user tilts backward.

The third region 1150 may be present in a third direction from the daily posture object 1120. The third region 1150 may be a region for representing that the user tilts in the third direction. For example, the third region 1150 may be a region for representing that the user tilts leftward.

The fourth region 1160 may be present in a fourth direction from the daily posture object 1120. The fourth region 1160 may be a region for representing that the user tilts in the fourth direction. For example, the fourth region 1160 may be a region for representing that the user tilts rightward.

FIG. 11 shows that the daily posture object 1120 includes four fan shapes, but the daily posture object 1120 is not limited thereto. Referring to FIG. 12, each of the first region 1130, the second region 1140, the third region 1150, and the fourth region 1160 may be divided into two subregions as shown in a daily posture object 1210.

However, the present disclosure is not limited thereto, and each of the first region 1130, the second region 1140, the third region 1150, and the fourth region 1160 may be divided into three subregions as shown in a daily posture object 1230. Also, each of the first region 1130, the second region 1140, the third region 1150, and the fourth region 1160 may be divided into two or more subregions.

Referring to FIGS. 10 and 11, the user terminal 100 may perform an operation 1020 of displaying at least one of the first region 1130, the second region 1140, the third region 1150, and the fourth region 1160 in a predetermined color on the basis of daily posture information. The daily posture information may include directional information and magnitude information of the user's tilt. For example, when the directional information of the user's tilt included in the daily posture information indicates the first direction, the user terminal 100 may display the first region 1130 in the predetermined color. Also, the predetermined color may be darker or stronger with an increase in the magnitude information of the user's tilt included in the daily posture information.

As described above, each of the first region 1130, the second region 1140, the third region 1150, and the fourth region 1160 may be divided into two or more subregions. For example, the first region 1130 may be divided into a 1-1 region 1221 and a 1-2 region 1222. With an increase in an angle at which the user's spine tilts, the posture correction system 200 may display that the angle is large in a farther region from the center of the circle. For example, when the user's spine tilts at a large angle in the first direction, the 1-2 region 1222 may be displayed in a dark or opaque color. The large angle at which the user's spine tilts in the first direction may represent that the angle is not included in the reference range information. Also, when the user's spine tilts at a small angle in the first direction, the 1-1 region 1221 may be displayed in a dark or opaque color. The small angle at which the user's spine tilts in the first direction may represent that the angle is included in the reference range information.

According to various embodiments of the present disclosure, the operation 320 of acquiring daily posture information may include the following operations. The user terminal 100 may display an object 1240 that is the user viewed from above. The user terminal 100 may perform an operation of displaying the daily posture object 1210 including at least one concentric circle around the object 1240 that is the user viewed from above. The concentric circle may surround 360 degrees of the object 1240. The concentric circle may indicate all directions including the forward, backward, left, and right directions of the user. The concentric circle may indicate an angle of the user's tilt. An outer concentric circle may be related to a larger tilt of the user. The user's tilt may be a spinal tilt of the user. The user's spine perpendicular to the ground may correspond to the maximum of the user's tilt, and an acute angle between the spine and the ground may correspond to the user's tilt.

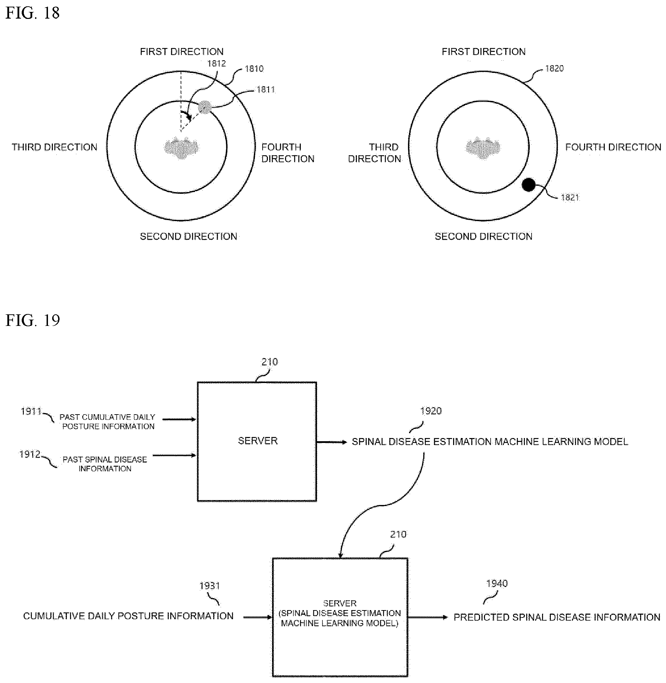

The user terminal 100 may perform an operation of displaying a color corresponding to the magnitude information included in the daily posture information at a position corresponding to the directional information included in the daily posture information in the daily posture object. Unlike FIG. 12, the daily posture object may not be divided into the first direction, the second direction, the third direction, and the fourth direction. The user terminal 100 may measure a tilt in 360-degree directions from the user. The directional information of the daily posture information may indicate one angle of 360 degrees around the user. The magnitude information of the daily posture information may indicate an angle at which the user's spine tilts. This will be described below with reference to FIG. 18.

FIG. 18 is a diagram illustrating a daily posture object according to an embodiment of the present disclosure.

Referring to a daily posture object 1810, the user terminal 100 may determine a position of an item 1811 on the basis of directional information 1812 included in daily posture information. Also, the user terminal 100 may determine which concentric circle the item 1811 will be disposed in on the basis of magnitude information included in the daily posture information. When the magnitude information is small, the item 1811 may be disposed in a circle close to the center of a concentric circle, and when the magnitude information is large, the item 1811 may be disposed in a circle far from the center of a concentric circle. Also, the user terminal 100 may determine a color of the item 1811 on the basis of one of a good posture time and a bad posture time based on cumulative daily posture information. When the item 1811 is related to a good posture, the user terminal 100 may refer to the good posture time. On the other hand, when the item 1811 is related to a bad posture, the user terminal 100 may refer to the bad posture time. In the daily posture object 1810, only the one item 1811 is shown, but the number of items shown in the daily posture object 1810 is not limited thereto. The user terminal 100 may display at least one item 1811 in the daily posture object 1810.

In addition, the item 1811 is only disposed on a concentric circle in the daily posture object 1810, but the position of an item is not limited thereto. In a daily posture object 1820, an item 1821 may be disposed between concentric circles. The distance from the center of a circle to the item 1821 may be directly proportional to magnitude information of daily posture information or cumulative posture information. The magnitude information is a continuous value and thus may be disposed between concentric circles.

The operation 1020 will be described in further detail below with reference to FIGS. 13 to 15.

FIG. 13 is a diagram illustrating an operation of the user terminal according to an embodiment of the present disclosure.

The operation 1020 of displaying at least one of the first to fourth regions 1130 to 1160 in a predetermined color shown in FIG. 10 may further include the following operations.

The directional information included in the daily posture information may indicate one of the forward, backward, left, and right directions. However, the directional information is not limited thereto and may have a value of 0 degrees to less than 360 degrees. In this case, the forward direction of the user may be assumed to be 0 degrees.

When the directional information is 315 degrees or more and less than 360 degrees or is 0 degrees or more and less than 45 degrees, the user terminal 100 may determine the user to be tilting forward. When the directional information is 45 degrees or more and less than 135 degrees, the user terminal 100 may determine the user to be tilting rightward. When the directional information is 135 degrees or more and less than 225 degrees, the user terminal 100 may determine the user to be tilting backward. When the directional information is 225 degrees or more and less than 315 degrees, the user terminal 100 may determine the user to be tilting leftward.

Referring to FIG. 13, when the daily posture information indicates that the user tilts forward, the user terminal 100 may perform an operation of displaying the first region 1130 in the predetermined color. With an increase in the user's tilt included in the daily posture information, the predetermined color may be displayed darker or more opaque.

When the daily posture information indicates that the user tilts backward, the user terminal 100 may perform an operation of displaying the second region 1140 in the predetermined color. With an increase in the user's tilt included in the daily posture information, the predetermined color may be displayed darker or more opaque.

When the daily posture information indicates that the user tilts leftward, the user terminal 100 may perform an operation of displaying the third region 1150 in the predetermined color. With an increase in the user's tilt included in the daily posture information, the predetermined color may be displayed darker or more opaque.

When the daily posture information indicates that the user tilts rightward, the user terminal 100 may perform an operation of displaying the fourth region 1160 in the predetermined color. With an increase in the user's tilt included in the daily posture information, the predetermined color may be displayed darker or more opaque.

According to various embodiments of the present disclosure, the user terminal may perform an operation of displaying at least one of the first region 1130, the second region 1140, the third region 1150, and the fourth region 1160 in a predetermined color on the basis of cumulative daily posture information.

More specifically, the user terminal 100 may perform an operation of acquiring cumulative daily posture information by accumulating daily posture information at predetermined intervals during a predetermined collection time. The user terminal 100 may perform an operation of acquiring cumulative daily posture information by accumulating daily posture information at predetermined intervals during a predetermined collection time. The predetermined collection time may be one of one day, one week, and one month. The predetermined intervals may be one second to one hour. For example, the user terminal 100 may acquire cumulative daily posture information of one day by accumulating daily posture information every minute.

The user terminal 100 may acquire a bad posture time from the cumulative daily posture information. The bad posture time may include at least one of a forward bad posture time, a backward bad posture time, a leftward bad posture time, and a rightward bad posture time. The bad posture time may indicate a time for which the user's tilt is maintained at an angle not included in the reference range information. The bad posture time may indicate a time for which the user is in an unhealthy posture. As described above, the cumulative daily posture information may be acquired at the predetermined intervals during the predetermined collection time, and when the user's tilt measured at one predetermined interval is not included in the reference range information, the user terminal 100 may determine the user to have stayed in a bad posture during the interval. The user terminal 100 may cumulatively measure a time for which the user is in a bad posture during the predetermined collection time. The user terminal 100 may cumulatively measure a time for which the user is in a bad posture for each piece of directional information. The user terminal 100 may acquire a time for which the user tilts forward while staying in a bad posture as a forward bad posture time. The user terminal 100 may acquire a time for which the user tilts backward while staying in a bad posture as a backward bad posture time. The user terminal 100 may acquire a time for which the user tilts leftward while staying in a bad posture as a leftward bad posture time. The user terminal 100 may acquire a time for which the user tilts rightward while staying in a bad posture as a rightward bad posture time.

The user terminal 100 may perform an operation of acquiring, as a forward bad posture time, a time with daily posture information which is not included in the reference range information and indicates that the user tilts forward in the cumulative daily posture information. The user terminal 100 may perform an operation of acquiring, as a backward bad posture time, a time with daily posture information which is not included in the reference range information and indicates that the user tilts backward in the cumulative daily posture information. The user terminal 100 may perform an operation of acquiring, as a leftward bad posture time, a time with daily posture information which is not included in the reference range information and indicates that the user tilts leftward in the cumulative daily posture information. The user terminal 100 may perform an operation of acquiring, as a rightward bad posture time, a time with daily posture information which is not included in the reference range information and indicates that the user tilts rightward in the cumulative daily posture information.

Referring to FIG. 13, the user terminal 100 may perform an operation of displaying the first region 1130 darker, stronger, or more opaque with an increase in the forward bad posture time. The user terminal 100 may perform an operation of displaying the second region 1140 darker, stronger, or more opaque with an increase in the backward bad posture time. The user terminal 100 may perform an operation of displaying the third region 1150 darker, stronger, or more opaque with an increase in the leftward bad posture time. The user terminal 100 may perform an operation of displaying the fourth region 1160 darker, stronger, or more opaque with an increase in the rightward bad posture time. Although it has been described above that the user terminal 100 displays the regions 1130, 1140, 1150, and 1160 darker, stronger, or more opaque with increases in the bad posture times, the present disclosure is not limited thereto. The user terminal 100 may display the regions 1130, 1140, 1150, and 1160 brighter, weaker, or more transparent with increases in the bad posture times. Also, the user terminal 100 may display bad posture times in the regions 1130, 1140, 1150, and 1160 as numbers.

As described above, the posture correction system 200 of the present disclosure shows a bad posture time for each direction, and thus it is possible to know which direction the user mainly tilts in. For example, in some cases, a user feels comfortable in a chair but is actually tilted in a certain direction, causing a constant force on his or her spine, resulting in disc symptoms. The posture correction system 200 of the present disclosure can analyze a user's long-term posture on the basis of cumulative daily posture information. Also, the posture correction system 200 can visually display a user's posture. In this way, a user can check his or her bad posture that he or she has not realized, and make efforts to correct the bad posture.

According to various embodiments of the present disclosure, the user terminal 100 may determine a color of the first region 1130 to the fourth region 1160 on the basis of the cumulative daily posture information in accordance with a time (frequency) corresponding to a tilt direction of the user. For example, with an increase in a time (frequency) indicating that the user tilts forward in the cumulative daily posture information, the first region 1130 may be displayed darker or more opaque. With an increase in a time (frequency) indicating that the user tilts backward in the cumulative daily posture information, the second region 1140 may be displayed darker or more opaque. With an increase in a time (frequency) indicating that the user tilts leftward in the cumulative daily posture information, the third region 1150 may be displayed darker or more opaque. With an increase in a time (frequency) indicating that the user tilts forward in the cumulative daily posture information, the fourth region 1160 may be displayed darker or more opaque.

More specifically, the user terminal 100 may perform an operation of acquiring cumulative daily posture information by accumulating daily posture information at the predetermined intervals during the predetermined collection time. The user terminal 100 may perform an operation of acquiring, as a forward posture time, a time with daily posture information indicating that the user tilts forward in the cumulative daily posture information. The user terminal 100 may perform an operation of acquiring, as a backward posture time, a time with daily posture information indicating that the user tilts backward in the cumulative daily posture information. The user terminal 100 may perform an operation of acquiring, as a leftward posture time, a time with daily posture information indicating that the user tilts leftward in the cumulative daily posture information. The user terminal 100 may perform an operation of acquiring, as a rightward posture time, a time with daily posture information indicating that the user tilts rightward in the cumulative daily posture information. The user

The user terminal 100 may perform an operation of displaying the first region darker, stronger, or more opaque with an increase in the forward posture time. The user terminal 100 may perform an operation of displaying the second region darker, stronger, or more opaque with an increase in the backward posture time. The user terminal 100 may perform an operation of displaying the third region darker, stronger, or more opaque with an increase in the leftward posture time. The user terminal 100 may perform an operation of displaying the fourth region darker, stronger, or more opaque with an increase in the rightward posture time. Although it has been described above that the user terminal 100 displays the regions 1130, 1140, 1150, and 1160 darker, stronger, or more opaque with increases in the posture times corresponding to directional information, the present disclosure is not limited thereto. The user terminal 100 may display the regions 1130, 1140, 1150, and 1160 brighter, weaker, or more transparent with increases in the posture times corresponding to directional information. Also, the user terminal 100 may display posture times corresponding to directional information in the regions 1130, 1140, 1150, and 1160 as numbers.

Referring to FIG. 13, cumulative posture information may indicate, for example, that a time for which the user tilts leftward is the longest, a time for which the user tilts forward or backward is moderate, and a time for which the user tilts rightward is the shortest. In this case, the user terminal 100 may display the third region 1150 as the darkest, the first and second regions 1130 and 1140 as moderately dark, and the fourth region 1160 as the brightest.

As described above, the posture correction system 200 of the present disclosure can display a posture that the user is mainly in. When the user constantly leans to one side, if not tilting his or her spine excessively, the user's spine may be strained. The posture correction system 200 of the present disclosure enables a user to check a posture that he or she is mainly in, causing the user to constantly care about his or her posture.

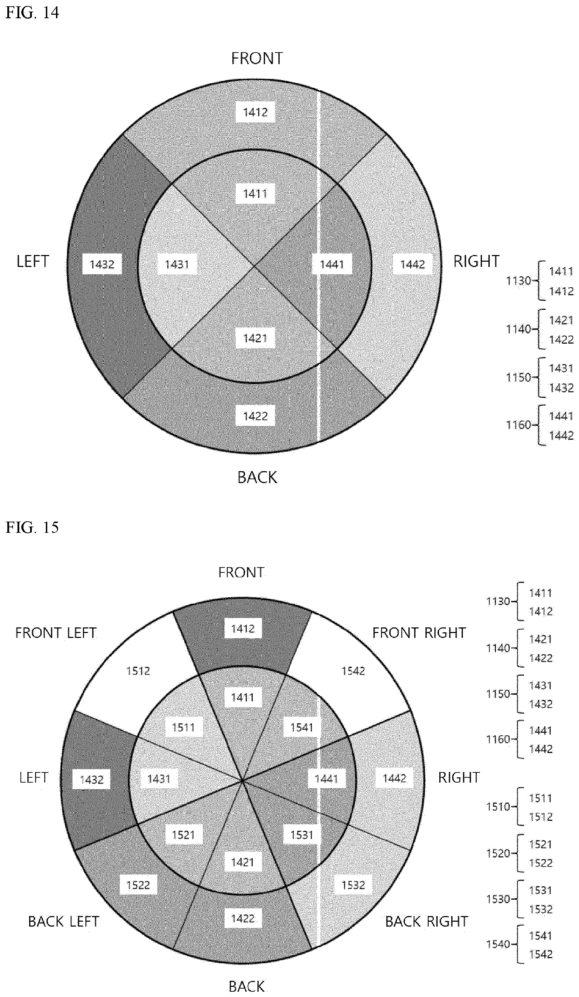

FIG. 14 is a diagram illustrating an operation of the user terminal according to an embodiment of the present disclosure.

As shown in FIG. 12, at least one of the first region 1130, the second region 1140, the third region 1150, and the fourth region 1160 may be divided into two or more subregions.

For example, the first region 1130 may include a 1-1 region 1411 and a 1-2 region 1412. The second region 1140 may include a 2-1 region 1421 and a 2-2 region 1422. The third region 1150 may include a 3-1 region 1431 and a 3-2 region 1432. The fourth region 1160 may include a 4-1 region 1441 and a 4-2 region 1442.

When daily posture information indicates that the user tilts forward and magnitude information of the tilt included in the daily posture information is included in the reference range information, the user terminal 100 may perform an operation of displaying the 1-1 region 1411 in the predetermined color. The 1-1 region 1411 may be displayed in the predetermined color when the user tilts forward but is in a good posture. When the magnitude information of the tilt included in the daily posture information is included in the reference range information, this may represent that the user tilts within a normal range.

When daily posture information indicates that the user tilts forward and magnitude information of the tilt included in the daily posture information is not included in the reference range information, the user terminal 100 may perform an operation of displaying the 1-2 region 1412 in the predetermined color. The 1-2 region 1412 may be displayed in the predetermined color when the user tilts forward and is in a bad posture. When the magnitude information of the tilt included in the daily posture information is not included in the reference range information, this may represent that the user excessively tilts. In other words, it may represent that the user is in a bad posture.

When daily posture information indicates that the user tilts backward and magnitude information of the tilt included in the daily posture information is included in the reference range information, the user terminal 100 may perform an operation of displaying the 2-1 region 1421 in the predetermined color. The 2-1 region 1421 may be displayed in the predetermined color when the user tilts backward but is in a good posture. When the magnitude information of the tilt included in the daily posture information is included in the reference range information, this may represent that the user tilts within a normal range.

When daily posture information indicates that the user tilts backward and magnitude information of the tilt included in the daily posture information is not included in the reference range information, the user terminal 100 may perform an operation of displaying the 2-2 region 1422 in the predetermined color. The 2-2 region 1422 may be displayed in the predetermined color when the user tilts backward and is in a bad posture. When the magnitude information of the tilt included in the daily posture information is not included in the reference range information, this may represent that the user excessively tilts. In other words, it may represent that the user is in a bad posture.

When daily posture information indicates that the user tilts leftward and magnitude information of the tilt included in the daily posture information is included in the reference range information, the user terminal 100 may perform an operation of displaying the 3-1 region 1431 in the predetermined color. The 3-1 region 1431 may be displayed in the predetermined color when the user tilts leftward but is in a good posture. When the magnitude information of the tilt included in the daily posture information is included in the reference range information, this may represent that the user tilts within a normal range.

When daily posture information indicates that the user tilts leftward and magnitude information of the tilt included in the daily posture information is not included in the reference range information, the user terminal 100 may perform an operation of displaying the 3-2 region 1432 in the predetermined color. The 3-2 region 1432 may be displayed in the predetermined color when the user tilts leftward and is in a bad posture. When the magnitude information of the tilt included in the daily posture information is not included in the reference range information, this may represent that the user excessively tilts. In other words, it may represent that the user is in a bad posture.

When daily posture information indicates that the user tilts rightward and magnitude information of the tilt included in the daily posture information is included in the reference range information, the user terminal 100 may perform an operation of displaying the 4-1 region 1441 in the predetermined color. The 4-1 region 1441 may be displayed in the predetermined color when the user tilts rightward but is in a good posture. When the magnitude information of the tilt included in the daily posture information is included in the reference range information, this may represent that the user tilts within a normal range.

When daily posture information indicates that the user tilts rightward and magnitude information of the tilt included in the daily posture information is not included in the reference range information, the user terminal 100 may perform an operation of displaying the 4-2 region 1442 in the predetermined color. The 4-2 region 1442 may be displayed in the predetermined color when the user tilts rightward and is in a bad posture. When the magnitude information of the tilt included in the daily posture information is not included in the reference range information, this may represent that the user excessively tilts. In other words, it may represent that the user is in a bad posture.

Referring to FIG. 14, since the 3-2 region 1432 displayed in the user terminal 100 is the darkest, it may be seen that the user is frequently in a leftward bad posture. Also, since the 4-1 region 1441 is the darkest among the 1-1 region 1411, the 2-1 region 1421, the 3-1 region 1431, and the 4-1 region 1441 displayed in the user terminal 100, it may be seen that the user mainly tilts rightward when in a good posture.

As described above, the posture correction system 200 of the present disclosure can determine which direction the user mainly tilts in while not only in a good posture but also in a bad posture. Accordingly, the user may make good efforts to correct his or her posture. Since it is not easy for the user to observe his or her own posture from the outside, it is difficult to know what posture he himself or she herself is in. However, the posture correction system 200 of the present disclosure provides various information to the user such that the user can objectively check his or her own posture.

Although the posture correction system 200 divides each of the regions 1130, 1140, 1150, and 1160 into two subregions in FIG. 14, the number of subregions is not limited thereto. The posture correction system 200 may divide each of the regions 1130, 1140, 1150, and 1160 into three or more subregions. The posture correction system 200 may represent that the user's tilt increases with an increase in the distance from the center of the circle. The posture correction system 200 may divide each of the regions 1130, 1140, 1150, and 1160 into three or more subregions on the basis of predetermined reference range information. Also, the posture correction system 200 may display the predetermined color in the regions 1130, 1140, 1150, and 1160 on the basis of at least one of daily posture information and cumulative daily posture information. This has been described above, and thus the description will not be reiterated.

FIG. 15 is a diagram illustrating an operation of the user terminal according to an embodiment of the present disclosure.

Unlike FIGS. 12 to 14, FIG. 15 shows a case where tilt direction information included in daily posture information includes forward, backward, left, right, forward left, backward left, forward right, and backward right directions. The first region 1130, the second region 1140, the third region 1150, and the fourth region 1160 have been described above, and thus the description will not be reiterated.

The user terminal 100 may further display a fifth region 1510, a sixth region 1520, a seventh region 1530, and an eighth region 1540. The user terminal 100 may divide each of the fifth region 1510, the sixth region 1520, the seventh region 1530, and the eighth region 1540 into at least one subregion. FIG. 15 shows a case where the user terminal 100 divides each of the fifth region 1510, the sixth region 1520, the seventh region 1530, and the eighth region 1540 into two subregions.

The fifth region 1510 may be related to a case where the directional information of the daily posture information indicates the forward left direction. The sixth region 1520 may be related to a case where the directional information of the daily posture information indicates the backward left direction. The seventh region 1530 may be related to a case where the directional information of the daily posture information indicates the backward right direction. The eighth region 1540 may be related to a case where the directional information of the daily posture information indicates the forward right direction.

The fifth region 1510 may include a 5-1 region 1511 and a 5-2 region 1512. The 5-1 region 1511 may be displayed in the predetermined color when the directional information of the daily posture information indicates the forward left direction and the magnitude information is included in the reference range information. The user terminal 100 may acquire a time for which the user tilts forward left while staying in a good posture on the basis of the cumulative daily posture information and display the 5-1 region 1511 in the predetermined color on the basis of the time.

The 5-2 region 1512 may be displayed in the predetermined color when the directional information of the daily posture information indicates the forward left direction and the magnitude information is not included in the reference range information. The user terminal 100 may acquire a time for which the user tilts forward left while staying in a bad posture on the basis of the cumulative daily posture information and display the 5-2 region 1512 in the predetermined color on the basis of the time.

A 6-1 region 1521 may be displayed in the predetermined color when the directional information of the daily posture information indicates the backward left direction and the magnitude information is included in the reference range information. The user terminal 100 may acquire a time for which the user tilts backward left while staying in a good posture on the basis of the cumulative daily posture information and display the 6-1 region 1521 in the predetermined color on the basis of the time.

A 6-2 region 1522 may be displayed in the predetermined color when the directional information of the daily posture information indicates the backward left direction and the magnitude information is not included in the reference range information. The user terminal 100 may acquire a time for which the user tilts backward left while staying in a bad posture on the basis of the cumulative daily posture information and display the 6-2 region 1522 in the predetermined color on the basis of the time.

A 7-1 region 1531 may be displayed in the predetermined color when the directional information of the daily posture information indicates the backward right direction and the magnitude information is included in the reference range information. The user terminal 100 may acquire a time for which the user tilts backward right while staying in a good posture on the basis of the cumulative daily posture information and display the 7-1 region 1531 in the predetermined color on the basis of the time.

A 7-2 region 1532 may be displayed in the predetermined color when the directional information of the daily posture information indicates the backward right direction and the magnitude information is not included in the reference range information. The user terminal 100 may acquire a time for which the user tilts backward right while staying in a bad posture on the basis of the cumulative daily posture information and display the 7-2 region 1532 in the predetermined color on the basis of the time.

An 8-1 region 1541 may be displayed in the predetermined color when the directional information of the daily posture information indicates the forward right direction and the magnitude information is included in the reference range information. The user terminal 100 may acquire a time for which the user tilts forward right while staying in a good posture on the basis of the cumulative daily posture information and display the 8-1 region 1541 in the predetermined color on the basis of the time.

An 8-2 region 1542 may be displayed in the predetermined color when the directional information of the daily posture information indicates the forward right direction and the magnitude information is not included in the reference range information. The user terminal 100 may acquire a time for which the user tilts forward right while staying in a bad posture on the basis of the cumulative daily posture information and display the 8-2 region 1542 in the predetermined color on the basis of the time.

In this way, the posture correction system 200 of the present disclosure can provide precise information to the user by displaying subdivided directional information of the user's tilt. Accordingly, the posture correction system 200 can precisely propose posture correction, and the user can precisely correct his or her posture.

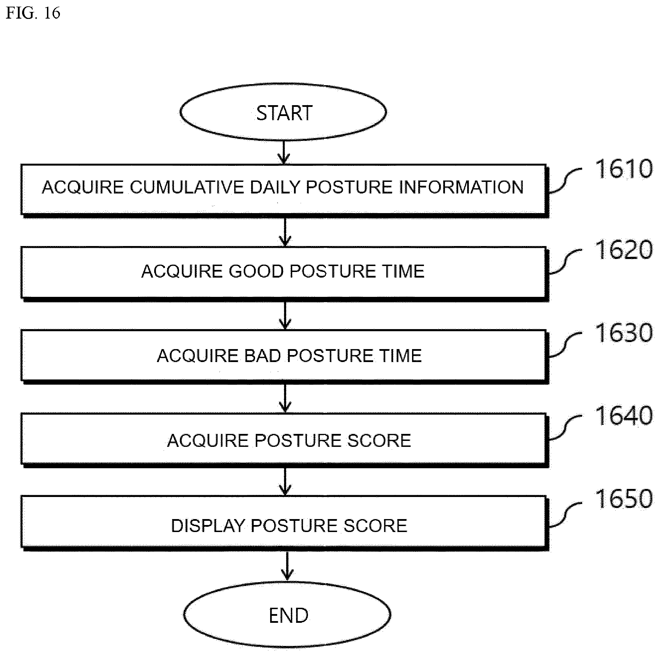

FIG. 16 is a flowchart illustrating operations of the posture correction system according to an embodiment of the present disclosure.

The user terminal 100 may perform an operation 1610 of acquiring cumulative daily posture information by accumulating daily posture information at the predetermined intervals during the predetermined collection time. This has been described above, and thus the description will not be reiterated.

The user terminal 100 may perform an operation 1620 of acquiring a time with daily posture information, which is included in the reference range information, in the cumulative daily posture information as a good posture time. As described above, the cumulative daily posture information may include a plurality of pieces of daily posture information which are periodically acquired. When magnitude information of one piece of daily posture information acquired at one predetermined interval among the plurality of pieces of daily posture information is included in the reference range information, the user terminal 100 may determine the user to have stayed in a good posture during the interval. The user terminal 100 may cumulatively measure a time for which the user is in a good posture during the predetermined collection time.

The user terminal 100 may perform an operation 1630 of acquiring a time with daily posture information, which is not included in the reference range information, in the cumulative daily posture information as a bad posture time. When magnitude information of one piece of daily posture information acquired at one predetermined interval among the plurality of pieces of daily posture information is not included in the reference range information, the user terminal 100 may determine the user to have stayed in a bad posture during the interval. The user terminal 100 may cumulatively measure a time for which the user is in a bad posture during the predetermined collection time.

The user terminal 100 may perform an operation 1640 of acquiring, as a posture score, a value calculated by dividing the good posture time by the collection time. However, a posture score is not limited thereto. The user terminal 100 may acquire a posture score by multiplying the value calculated by dividing the good posture time by the collection time by 100. The user terminal 100 may acquire a posture score that increases with an increase in the good posture time using a different method.

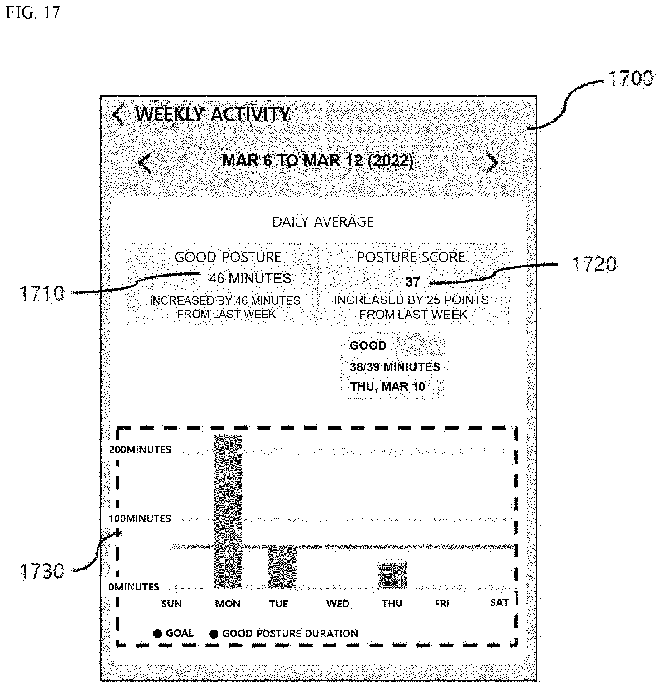

Also, the user terminal 100 may perform an operation 1650 of displaying the posture score. The operation 1650 will be described with reference to FIG. 17.

FIG. 17 shows a screen of the user terminal according to an embodiment of the present disclosure.

The user terminal 100 may display a screen 1700. The screen 1700 may be a screen on which daytime statistics are displayed. In other words, the screen 1700 shows a case where the collection time is one week.

The user terminal 100 may display a good posture time 1710. The good posture time 1710 may be a value calculated by dividing a good posture time accumulated during one week by seven days. Also, the good posture time 1710 may indicate an increase in the good posture time from the previous week.

The good posture time may be information acquired in the operation 1620. The user terminal 100 may display a posture score 1720. The posture score 1720 shows a digitized time for which the user is in a good posture but does not indicate how long the user has actually stayed in a good posture. Accordingly, the user terminal 100 may display not only the good posture time 1710 but also the posture score 1720 on one screen at the same time.

The user terminal 100 may acquire a posture score on the basis of a good posture time in one week. However, the present disclosure is not limited thereto, and the user terminal 100 may acquire a posture score on the basis of an everyday good posture time and display an average of posture scores that are acquired on a daily basis on the screen 1700.

The user terminal 100 may display the good posture time as a graph 1730. The graph 1730 may show the user's good posture time by day of the week.

Since the posture correction system of the present disclosure shows whether a user's posture is good using a posture score, the user can make efforts to increase the posture score. Also, the posture score will be increased according to the user's efforts, and thus the user will be motivated to stay in a good posture. Accordingly, the user may utilize the posture correction system 200 for a long time.

At least one of the user terminal 100 and the posture sensor 220 of the present disclosure may perform an operation of acquiring the user's posture pattern. The posture pattern may include at least one of a standing pose, a squatting pose, a chair pose, a walking pose, and a running pose. The posture pattern may include a plurality of exercise postures. For example, the posture pattern may include squats, sit-ups, Pilates, swimming, running, and the like. At least one of the user terminal 100 and the posture sensor 220 may determine the user's current posture pattern on the basis of the inertial sensor.

The user terminal 100 may select at least one of reference range information and a posture estimation machine learning model corresponding to the posture pattern. The reference range information may vary depending on the user's posture pattern. In other words, depending on whether the user is standing or sitting, a good posture may be determined differently. The user terminal 100 may determine that a good posture differs in accordance with a posture pattern by determining reference range information on the basis of the user's current posture pattern. Selecting reference range information on the basis of a posture pattern may result in an improvement in performance of the posture correction system 200.

As described above, a posture estimation machine learning model may be an element for outputting 3D posture information on the basis of posture image information. Performance of a posture estimation machine learning model may vary depending on a posture pattern. The user terminal 100 or the server 210 may select a posture estimation machine learning model in accordance with the user's current posture pattern. Accordingly, the user terminal 100 or the server 210 can utilize a posture estimation machine learning model optimized for a posture pattern. Consequently, the performance of the posture correction system 200 can be improved.

FIG. 19 is a diagram illustrating an operation of the posture correction system according to an embodiment of the present disclosure.

The posture correction system 200 may further include a spinal disease estimation machine learning model 1920. The spinal disease estimation machine learning model 1920 may be a machine learning model for predicting spinal disease information from cumulative daily posture information. The spinal disease estimation machine learning model 1920 may be a model for selecting one of a plurality of spinal diseases on the basis of cumulative daily posture information. The spinal disease estimation machine learning model 1920 may be implemented on the basis of a machine learning model based on a CNN or transformer. The spinal disease estimation machine learning model 1920 may utilize past cumulative daily posture information 1911 and past spinal disease information 1912 as training data. The past cumulative daily posture information 1911 may be a target of analysis, and the past spinal disease information 1912 may be label information acquired by a person or device. The past spinal disease information 1912 may be a ground truth value. The past cumulative daily posture information 1911 and the past spinal disease information 1912 may correspond to each other on a one-to-one basis. The posture correction system 200 may collect the past cumulative daily posture information 1911 from a plurality of users. Also, the posture correction system 200 may acquire, as the past spinal disease information 1912, information on spinal diseases developing in the users corresponding to the past cumulative daily posture information 1911 within a predetermined analysis time. The predetermined analysis time may be one day or more and one year or less. The spinal disease information may be identification information of a spinal disease, for example, disease code of a spinal disease.

In addition to the past cumulative daily posture information 1911, at least one of past good posture times or past bad posture times may be used for machine learning. However, the present disclosure is not limited thereto, and at least one of past good posture times or past bad posture times may be used instead of the past cumulative daily posture information 1911 for machine learning. At least one of past good posture times or past bad posture times may include tilt directions and times for which tilts are maintained.

The server 210 may update the spinal disease estimation machine learning model 1920 using forward propagation and backpropagation. The server 210 may perform machine learning on correlation of the past spinal disease information 1912 with the past cumulative daily posture information 1911. When the accuracy of the spinal disease estimation machine learning model 1920 is sufficiently high, the server 210 may stop updating the spinal disease estimation machine learning model 1920. The server 210 may transmit the spinal disease estimation machine learning model 1920 to the user terminal 100 or another server. The server 210 may store the spinal disease estimation machine learning model 1920.

The server 210 may receive cumulative daily posture information 1931 of a current user from the user terminal 100 or the posture sensor 220. A process of acquiring cumulative daily posture information 1931 has been described above, and thus the description will not be reiterated. The server 210 may perform an operation of acquiring spinal disease information 1940 predicted by applying the cumulative daily posture information 1931 to the spinal disease estimation machine learning model 1920. The spinal disease information 1940 acquired by applying the cumulative daily posture information 1931 to the spinal disease estimation machine learning model 1920 may be predicted information. The spinal disease information 1940 predicted by the spinal disease estimation machine learning model 1920 may be almost the same as actual spinal disease information.

Result information output by the spinal disease estimation machine learning model 1920 may be a disease possibility of each of a plurality of spinal diseases. In other words, the server 210 may perform an operation of acquiring a disease possibility of each of a plurality of spinal diseases by applying cumulative daily posture information to the spinal disease estimation machine learning model.

The posture correction system 200 may select a disease possibility that is a predetermined threshold possibility or more and the highest value among the disease possibilities of the plurality of spinal diseases. The posture correction system 200 may select a spine disease corresponding to the highest possibility value and determine the spine disease as the spinal disease information 1940. The posture correction system 200 may output the possibility value corresponding to the spinal disease information 1940. When the possibility value corresponding to the spinal disease information 1940 is very high, it is highly likely that the user will develop a disease corresponding to the spinal disease information 1940 within the analysis time. Also, when the possibility value corresponding to the spinal disease information 1940 is relatively low, it is unlikely that the user will develop the disease corresponding to the spinal disease information 1940 within the analysis time. The user may be motivated to correct his or her posture by checking the possibility of developing the disease corresponding to the spinal disease information 1940.

A process of determining whether a user stays in a healthy posture for each body part using 3D posture information will be described below. When the posture correction system 200 determines whether the user is following the reference posture information based on the exercise information in operation 360, the posture correction system 200 determines whether the user is in a healthy and good posture for each body part in the following operations.

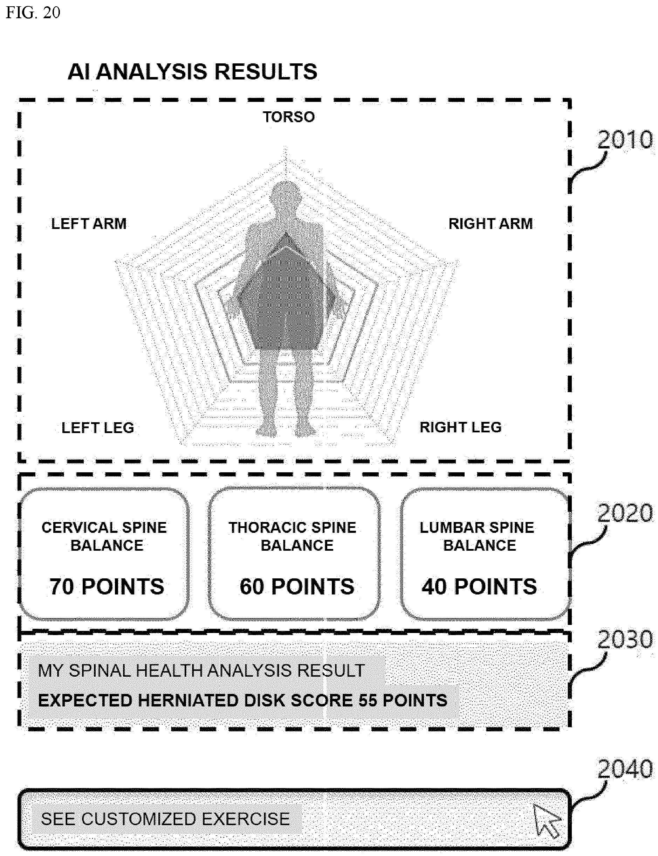

FIG. 20 is a diagram illustrating an operation of the user terminal according to an embodiment of the present disclosure.

At least one of the server 210 and the user terminal 100 may perform an operation of determining posture similarity between 3D posture information and predetermined good posture information. The operation of determining posture similarity may be similar to the operation 360.

At least one of the server 210 and the user terminal 100 may determine whether 3D posture information corresponds to good posture information for each predetermined body part of the user. The predetermined good posture information may be information for determining whether the user is in a healthy posture for a predetermined body part. With an increase in the similarity between 3D posture information and good posture information, the posture correction system 200 may increase a health score of a corresponding body part. However, the present disclosure is not limited thereto, and the posture correction system 200 may utilize predetermined bad posture information instead of good posture information. With an increase in the similarity between 3D posture information and bad posture information, the posture correction system 200 may reduce a health score of a corresponding body part. Good posture information or bad posture information may be determined by a professional such as a medical worker or trainer.

The predetermined body part may include at least one of the torso, the left arm, the right arm, the left leg, and the right leg. For example, when the user is sitting and keeping his or her right shoulder higher than the left shoulder, the posture correction system 200 may reduce the health score of the left or right arm. Also, when the user is sitting and crossing his or her legs with the right leg over the left leg, the posture correction system 200 may reduce the health score of the left or right leg. Further, when the user excessively tilts his or her spine with respect to the ground, the posture correction system 200 may reduce the health score of the torso.

The posture correction system 200 may determine at least one of good posture information and bad posture information on the basis of a posture pattern. A method of determining a posture pattern has already been described above, and the description will not be reiterated. Depending on a posture pattern, a good posture and a bad posture may vary. For example, when a posture pattern is squatting, sitting in a squat with the user's knees in front of the feet may be bad for the legs. On the other hand, when the user is sitting, the user's knees in front of the feet may not be problematic at all.

As described above, 3D posture information may include identification information corresponding to body feature nodes and angles of joints corresponding to the identification information. Good posture information may also include identification information corresponding to body feature nodes and angles of joints corresponding to the identification information. The server 210 may determine posture similarity between joint angles of 3D posture information and reference posture information having the same identification information. For example, the posture similarity may be determined to be a difference ratio in accordance with the following equation.

Difference ratio = |joint angle of 3D posture information-joint angle of good posture information|/joint angle of good posture information

When a difference ratio is smaller, a health score of the predetermined body part may be higher. The posture correction system 200 may acquire a health score for each predetermined body part. The user terminal 100 may display a graph showing health scores of body parts in one region 2010 of the screen. For example, with an increase in the distance from the center of the graph, a health score of a corresponding body part may be higher.

According to various embodiments of the present disclosure, the posture correction system 200 may acquire 3D posture information at predetermined intervals during a collection time. When a difference ratio of one of a plurality of pieces of 3D posture information is a predetermined threshold ratio or less, the posture correction system 200 may determine that the 3D posture information corresponds to good posture information. The posture correction system 200 may determine whether 3D posture information corresponds to good posture information for each of predetermined body parts. Also, when 3D posture information corresponds to good posture information, the posture correction system 200 may determine that the user has stayed in a good posture during an interval corresponding to the 3D posture information. The posture correction system 200 may acquire a good posture time, which is a time for which the user stays in a good posture, for each predetermined body part on the basis of a plurality of pieces of 3D posture information during the collection time. The posture correction system 200 may determine a score on the basis of a value calculated by dividing a good posture time by the collection time. Also, the user terminal 100 may display a graph showing a health score of each body part in the region 2010 of the screen.

In the above description, a health score is acquired using good posture information, but the posture correction system 100 may utilize bad posture information.

Difference ratio = |joint angle of 3D posture information-joint angle of bad posture information|/joint angle of bad posture information

In this case, when a difference ratio is smaller, a health score of the predetermined body part may be lower. The user terminal 200 may display a graph showing health scores of body parts in the region 2010 of the screen.

As described above, the posture correction system 200 may acquire a posture score for the spine. The posture correction system 200 may divide the spine into cervical, thoracic, and lumbar vertebrae and acquire a posture score for each of the vertebrae. The posture correction system 200 may determine a good posture time by comparing 3D posture information with good posture information. The posture correction system 200 may determine a good posture time for at least one of the cervical, thoracic, and lumbar vertebrae. A good posture time may be a time for which 3D posture information corresponds to good posture information. Correspondence may represent a case where a difference ratio based on 3D posture information and good posture information is a predetermined threshold difference or less. However, a good posture time is not limited thereto and may also be a time for which 3D posture information does not correspond to bad posture information.

The posture correction system 200 may acquire a value calculated by dividing a good posture time by the collection time as a posture score. The posture score may include at least one of cervical, thoracic, and lumbar vertebra posture scores. The user terminal 100 may display at least one of the cervical, thoracic, and lumbar vertebra posture scores in a region 2020. Also, the user terminal 100 may display a posture score of the spine in a region 2030. The posture score of the spine may be the posture score acquired in the operation 1640.

The posture correction system 200 may recommend exercise information on the basis of at least one of the posture score of the spine, the cervical vertebra posture score, the thoracic vertebra posture score, the lumbar vertebra posture score, and health scores of predetermined body parts. More specifically, the posture correction system 200 may store the exercise information selection table in which exercise information corresponds to at least one of the posture score of the spine, the cervical vertebra posture score, the thoracic vertebra posture score, the lumbar vertebra posture score, and the health scores of the predetermined body parts. The posture correction system may select exercise information corresponding to at least one of the posture score of the spine, the cervical vertebra posture score, the thoracic vertebra posture score, the lumbar vertebra posture score, and the health scores of the predetermined body parts from the exercise information selection table. The user may check the exercise information by making an input of a button 2040 to the user terminal 100.

As described above, the posture correction system of the present disclosure can display a score not only for a user's spine but also for each body part, and thus the user can check his or her physical health by body part. Also, the user can check a part that he or she should care about for health, and try to make the part healthier. Therefore, the user can be aware of the part that he or she should focus on to stay healthy, and put no resources into unnecessary parts. Consequently, the posture correction system of the present disclosure can remarkably improve the user's health.

The present invention has been described with reference to various embodiments. Those of ordinary skill in the art to which the present invention pertains should understand that the present invention can be implemented in modified forms without departing from the essential characteristics of the present invention. Therefore, the disclosed embodiments should be considered in a descriptive sense rather than a restrictive sense. The scope of the present invention is not described above, but in the claims.

Meanwhile, the above-described embodiments of the present invention can be written as a computer-executable program and may be implemented in a general-use digital computer that executes the program using a computer-readable recording medium. The computer-readable recording medium is a storage medium such as a magnetic storage medium (e.g., a ROM, a floppy disk, a hard disk drive (HDD), or the like) or an optical medium (e.g., a compact disc (CD)-ROM, a digital versatile disc (DVD), or the like).

**Claims**

1. An operating method of a posture correction system for correcting a posture using artificial intelligence (AI), wherein the posture correction system includes a server (210), a user terminal (100) and a posture sensor (220), the operating method comprising:

   setting, by the user terminal, reference range information of a good posture on the basis of an input of a user;
   acquiring daily posture information of the user on the basis of a daily posture sensor which is included in a posture sensor and measures a tilt of the user's spine;
   outputting, by the user terminal, a message recommending straightening up a posture when the daily posture information is not included in the reference range information;
   acquiring posture image information of the user on the basis of an exercise posture sensor which is included in the posture sensor and images the user's entire body;
   acquiring, by the server, three-dimensional (3D) posture information by applying the posture image information and the daily posture information to a posture estimation machine learning model;
   determining, by the server, posture similarity between the 3D posture information and predetermined reference posture information; and
   outputting, by the user terminal, a message recommending correcting the posture when the posture similarity is less than predetermined threshold similarity.

2. The operating method of claim 1, wherein the setting of the reference range information comprises:

   receiving a user input related to the reference range information from the user;
   determining, by the user terminal, predetermined initial range information as the reference range information when the user input indicates automatic range setting;
   displaying, by the user terminal, a user interface for range setting when the user input indicates manual range setting;
   acquiring, by the user terminal, the tilt of the user's spine on the basis of the daily posture sensor;
   changing, by the user terminal, a tilt of a body object representing at least a part of the body included in the user interface on the basis of the tilt of the spine and outputting the body object; and
   determining, by the user terminal, 0 degrees to the tilt of the spine as the reference range information when the user confirms the tilt of the body object and makes an input of an object representing confirmation.

3. The operating method of claim 1, wherein the acquiring of the daily posture information comprises:

   displaying a daily posture object including a first region, a second region, a third region, and a fourth region; and
   displaying at least one of the first to fourth regions in a predetermined color on the basis of the daily posture information.

4. The operating method of claim 3, wherein the displaying of the at least one of the first to fourth regions in the predetermined color comprises:

   displaying the first region in the predetermined color when the daily posture information indicates that the user tilts forward;
   displaying the second region in the predetermined color when the daily posture information indicates that the user tilts backward;
   displaying the third region in the predetermined color when the daily posture information indicates that the user tilts leftward;
   displaying the fourth region in the predetermined color when the daily posture information indicates that the user tilts rightward.

5. The operating method of claim 3, wherein the first region includes a 1-1 region and a 1-2 region,

the second region includes a 2-1 region and a 2-2 region,
the third region includes a 3-1 region and a 3-2 region,
the fourth region includes a 4-1 and a 4-2 region, and
the operating method further comprises:

displaying the 1-1 region in the predetermined color when the daily posture information indicates that the user tilts forward and magnitude information of a tilt included in the daily posture information is included in the reference range information;
displaying the 1-2 region in the predetermined color when the daily posture information indicates that the user tilts forward and the magnitude information of the tilt included in the daily posture information is not included in the reference range information;
displaying the 2-1 region in the predetermined color when the daily posture information indicates that the user tilts backward and the magnitude information of the tilt included in the daily posture information is included in the reference range information;
displaying the 2-2 region in the predetermined color when the daily posture information indicates that the user tilts backward and the magnitude information of the tilt included in the daily posture information is not included in the reference range information;
displaying the 3-1 region in the predetermined color when the daily posture information indicates that the user tilts leftward and the magnitude information of the tilt included in the daily posture information is included in the reference range information;
displaying the 3-2 region in the predetermined color when the daily posture information indicates that the user tilts leftward and the magnitude information of the tilt included in the daily posture information is not included in the reference range information;
displaying the 4-1 region in the predetermined color when the daily posture information indicates that the user tilts rightward and the magnitude information of the tilt included in the daily posture information is included in the reference range information; and
displaying the 4-2 region in the predetermined color when the daily posture information indicates that the user tilts rightward and the magnitude information of the tilt included in the daily posture information is not included in the reference range information.

6. The operating method of claim 3, comprising:

acquiring, by the user terminal, cumulative daily posture information by accumulating the daily posture information at predetermined intervals during a predetermined collection time;
acquiring a time with daily posture information, which is not included in the reference range information and indicates that the user tilts forward, in the cumulative daily posture information as a bad forward posture time;
acquiring a time with daily posture information, which is not included in the reference range information and indicates that the user tilts backward, in the cumulative daily posture information as a bad backward posture time;
acquiring a time with daily posture information, which is not included in the reference range information and indicates that the user tilts leftward, in the cumulative daily posture information as a bad leftward posture time;
acquiring a time with daily posture information, which is not included in the reference range information and indicates that the user tilts rightward, in the cumulative daily posture information as a bad rightward posture time;
displaying the first region darker, stronger, or more opaque with an increase in the bad forward posture time;
displaying the second region darker, stronger, or more opaque with an increase in the bad backward posture time;
displaying the third region darker, stronger, or more opaque with an increase in the bad leftward posture time; and
displaying the fourth region darker, stronger, or more opaque with an increase in the bad rightward posture time.

7. The operating method of claim 3, comprising:

acquiring, by the user terminal, cumulative daily posture information by accumulating the daily posture information at predetermined intervals during a predetermined collection time;
acquiring a time with daily posture information, which indicates that the user tilts forward, in the cumulative daily posture information as a forward posture time;
acquiring a time with daily posture information, which indicates that the user tilts backward, in the cumulative daily posture information as a backward posture time;
acquiring a time with daily posture information, which indicates that the user tilts leftward, in the cumulative daily posture information as a leftward posture time;

acquiring a time with daily posture information, which indicates that the user tilts rightward, in the cumulative daily posture information as a rightward posture time;

displaying the first region darker, stronger, or more opaque with an increase in the forward posture time;

displaying the second region darker, stronger, or more opaque with an increase in the backward posture time;

displaying the third region darker, stronger, or more opaque with an increase in the leftward posture time; and

displaying the fourth region darker, stronger, or more opaque with an increase in the rightward posture time.

8. The operating method of claim 1, comprising:

acquiring, by the user terminal, cumulative daily posture information by accumulating the daily posture information at predetermined intervals during a predetermined collection time;

acquiring a time with daily posture information, which is included in the reference range information, in the cumulative daily posture information as a good posture time;

acquiring a time with daily posture information, which is not included in the reference range information, in the cumulative daily posture information as a bad posture time;

acquiring a value calculated by dividing the good posture time by the collection time as a posture score; and

displaying the posture score.

9. The operating method of claim 1, comprising:

acquiring, by at least one of the user terminal and the posture sensor, a posture pattern of the user; and

selecting at least one of reference range information and the posture estimation machine learning model corresponding to the posture pattern,

wherein the posture pattern includes at least one of a standing pose, a sitting pose, a walking pose, and a running pose.

10. The operating method of claim 1, wherein the 3D posture information includes a plurality of body feature nodes which correspond to at least some joints of the body and skeletal edges connecting the plurality of body feature nodes, and the acquiring of the 3D posture information comprises causing an angle of a skeletal edge, which is included in the 3D posture information and corresponds to the spine, with respect to a ground surface to correspond to the daily posture information.

11. The operating method of claim 1, comprising:

selecting, by the user terminal, exercise information beneficial to the user from among a plurality of pieces of candidate exercise information on the basis of the daily posture information;

outputting, by the user terminal, the exercise information; and

acquiring, by the user terminal, the posture image information of the user on the basis of the exercise posture sensor when the user takes a posture on the basis of the exercise information.

12. The operating method of claim 11, wherein the selecting of the exercise information comprises:

acquiring, by the user terminal, disease information from the user;

selecting partial candidate exercise information by excluding exercise information harmful to the user from the plurality of pieces of candidate exercise information on the basis of the disease information; and

selecting the exercise information from the partial candidate exercise information on the basis of the daily posture information.

13. The operating method of claim 1, wherein the acquiring of the daily posture information comprises:

displaying a daily posture object including at least one concentric circle around an object which is the user seen from above; and

displaying a color corresponding to magnitude information included in the daily posture information at a position corresponding to directional information included in the daily posture information in the daily posture object.

14. The operating method of claim 1, wherein the determining, by the user terminal, of the posture similarity between the 3D posture information and the predetermined reference posture information comprises:

determining whether the 3D posture information corresponds to good posture information for each predetermined body part of the user;

increasing a health score of a corresponding part with an increase in similarity between the 3D posture information and the good posture information; and

displaying a graph showing health scores of the body parts in an area of a screen.

**Patentansprüche**

1. Betriebsverfahren eines Haltungskorrektursystems zum Korrigieren einer Haltung unter Verwendung von künstlicher Intelligenz, KI, wobei das Haltungskorrektursystem einen Server (210), ein Benutzerendgerät (100) und einen Haltungssensor (220) umfasst, wobei das Betriebsverfahren umfasst:

Einstellen, durch das Benutzerendgerät, von Referenzbereichsinformationen einer guten Haltung auf der Grundlage einer Eingabe eines Benutzers;

Erfassen von täglichen Haltungsinformationen des Benutzers auf der Grundlage eines täglichen Haltungssensors, der in einem Haltungssensor enthalten ist und eine Neigung der Wirbelsäule des Benutzers misst;

Ausgeben, durch das Benutzerendgerät, einer Nachricht, die empfiehlt, eine Haltung aufzurichten, wenn die täglichen Haltungsinformationen nicht in den Referenzbereichsinformationen enthalten sind;

Erfassen von Haltungsbildinformationen des Benutzers auf der Grundlage eines Übungshaltungssensors, der in dem Haltungssensor enthalten ist und den gesamten Körper des Benutzers abbildet;

Erfassen, durch den Server, von dreidimensionalen, 3D-, Haltungsinformationen durch Anwenden der Haltungsbildinformationen und der täglichen Haltungsinformationen auf ein Modell für maschinelles Lernen zur Haltungsschätzung;

Bestimmen, durch den Server, einer Haltungsähnlichkeit zwischen den 3D-Haltungsinformationen und vorbestimmten Referenzhaltungsinformationen; und

Ausgeben, durch das Benutzerendgerät, einer Nachricht, die das Korrigieren der Haltung empfiehlt, wenn die Haltungsähnlichkeit geringer ist als eine vorbestimmte Schwellenwertähnlichkeit.

2. Betriebsverfahren nach Anspruch 1, wobei das Einstellen der Referenzbereichsinformationen umfasst:

Empfangen einer Benutzereingabe, die sich auf die Referenzbereichsinformationen bezieht, von dem Benutzer;

Bestimmen, durch das Benutzerendgerät, von vorbestimmten anfänglichen Bereichsinformationen als die Referenzbereichsinformationen, wenn die Benutzereingabe eine automatische Bereichseinstellung angibt;

Anzeigen, durch das Benutzerendgerät, einer Benutzerschnittstelle für eine Bereichseinstellung, wenn die Benutzereingabe eine manuelle Bereichseinstellung angibt;

Erfassen, durch das Benutzerendgerät, der Neigung der Wirbelsäule des Benutzers auf der Grundlage des täglichen Haltungssensors;

Ändern, durch das Benutzerendgerät, einer Neigung eines Körperobjekts, das mindestens einen Teil des in der Benutzerschnittstelle enthaltenen Körpers darstellt, auf der Grundlage der Neigung der Wirbelsäule, und Ausgeben des Körperobjekts; und

Bestimmen, durch das Benutzerendgerät, einer Neigung der Wirbelsäule von 0 Grad als die Referenzbereichsinformationen, wenn der Benutzer die Neigung des Körperobjekts bestätigt und eine Eingabe eines Objekts macht, das die Bestätigung darstellt.

3. Betriebsverfahren nach Anspruch 1, wobei das Erfassen der täglichen Haltungsinformationen umfasst:

Anzeigen eines täglichen Haltungsobjekts, das einen ersten Bereich, einen zweiten Bereich, einen dritten Bereich und einen vierten Bereich umfasst; und

Anzeigen von mindestens einem der ersten bis vierten Bereiche in einer vorbestimmten Farbe auf der Grundlage der täglichen Haltungsinformationen.

4. Betriebsverfahren nach Anspruch 3, wobei das Anzeigen des mindestens einen der ersten bis vierten Bereiche in der vorbestimmten Farbe umfasst:

Anzeigen des ersten Bereichs in der vorbestimmten Farbe, wenn die täglichen Haltungsinformationen angeben, dass sich der Benutzer nach vorne neigt;

Anzeigen des zweiten Bereichs in der vorbestimmten Farbe, wenn die täglichen Haltungsinformationen angeben, dass sich der Benutzer nach hinten neigt;

Anzeigen des dritten Bereichs in der vorbestimmten Farbe, wenn die täglichen Haltungsinformationen angeben, dass sich der Benutzer nach links neigt;

Anzeigen des vierten Bereichs in der vorbestimmten Farbe, wenn die täglichen Haltungsinformationen angeben, dass sich der Benutzer nach rechts neigt.

5. Betriebsverfahren nach Anspruch 3, wobei der erste Bereich einen 1-1-Bereich und einen 1-2-Bereich umfasst,

der zweite Bereich einen 2-1-Bereich und einen 2-2-Bereich umfasst,
der dritte Bereich einen 3-1-Bereich und einen 3-2-Bereich umfasst,
der vierte Bereich einen 4-1-Bereich und einen 4-2-Bereich umfasst, und
das Betriebsverfahren ferner umfasst:

Anzeigen des 1-1-Bereichs in der vorbestimmten Farbe, wenn die täglichen Haltungsinformationen angeben, dass sich der Benutzer nach vorne neigt und in den täglichen Haltungsinformationen enthaltene Größeninformationen einer Neigung in den Referenzbereichsinformationen enthalten sind;

Anzeigen des 1-2-Bereichs in der vorbestimmten Farbe, wenn die täglichen Haltungsinformationen angeben, dass sich der Benutzer nach vorne neigt und die in den täglichen Haltungsinformationen enthaltenen Größeninformationen der Neigung nicht in den Referenzbereichsinformationen enthalten sind;

Anzeigen des 2-1-Bereichs in der vorbestimmten Farbe, wenn die täglichen Haltungsinformationen angeben, dass sich der Benutzer nach hinten neigt und die in den täglichen Haltungsinformationen enthaltenen Größeninformationen der Neigung in den Referenzbereichsinformationen enthalten sind;

Anzeigen des 2-2-Bereichs in der vorbestimmten Farbe, wenn die täglichen Haltungsinformationen angeben, dass sich der Benutzer nach hinten neigt und die in den täglichen Haltungsinformationen enthaltenen Größeninformationen der Neigung nicht in den Referenzbereichsinformationen enthalten sind;

Anzeigen des 3-1-Bereichs in der vorbestimmten Farbe, wenn die täglichen Haltungsinformationen angeben, dass sich der Benutzer nach links neigt und die in den täglichen Haltungsinformationen enthaltenen Größeninformationen der Neigung in den Referenzbereichsinformationen enthalten sind;

Anzeigen des 3-2-Bereichs in der vorbestimmten Farbe, wenn die täglichen Haltungsinformationen angeben, dass sich der Benutzer nach links neigt und die in den täglichen Haltungsinformationen enthaltenen Größeninformationen der Neigung nicht in den Referenzbereichsinformationen enthalten sind;

Anzeigen des 4-1-Bereichs in der vorbestimmten Farbe, wenn die täglichen Haltungsinformationen angeben, dass sich der Benutzer nach rechts neigt und die in den täglichen Haltungsinformationen enthaltenen Größeninformationen der Neigung in den Referenzbereichsinformationen enthalten sind; und

Anzeigen des 4-2-Bereichs in der vorbestimmten Farbe, wenn die täglichen Haltungsinformationen angeben, dass sich der Benutzer nach rechts neigt und die in den täglichen Haltungsinformationen enthaltenen Größeninformationen der Neigung nicht in den Referenzbereichsinformationen enthalten sind.

6. Betriebsverfahren nach Anspruch 3, umfassend:

Erfassen, durch das Benutzerendgerät, von kumulativen täglichen Haltungsinformationen durch Akkumulieren der täglichen Haltungsinformationen in vorbestimmten Intervallen während einer vorbestimmten Sammelzeit;

Erfassen einer Zeit mit täglichen Haltungsinformationen, die nicht in den Referenzbereichsinformationen enthalten sind und angeben, dass sich der Benutzer nach vorne neigt, in den kumulativen täglichen Haltungsinformationen als eine schlechte Vorwärtshaltungszeit;

Erfassen einer Zeit mit täglichen Haltungsinformationen, die nicht in den Referenzbereichsinformationen enthalten sind und angeben, dass sich der Benutzer nach hinten neigt, in den kumulativen täglichen Haltungsinformationen als eine schlechte Rückwärtshaltungszeit;

Erfassen einer Zeit mit täglichen Haltungsinformationen, die nicht in den Referenzbereichsinformationen enthalten sind und angeben, dass sich der Benutzer nach links neigt, in den kumulativen täglichen Haltungsinformationen als eine schlechte Linkshaltungszeit;

Erfassen einer Zeit mit täglichen Haltungsinformationen, die nicht in den Referenzbereichsinformationen enthalten sind und angeben, dass sich der Benutzer nach rechts neigt, in den kumulativen täglichen Haltungsinformationen als eine schlechte Rechtshaltungszeit;

Anzeigen des ersten Bereichs dunkler, stärker oder opaker mit einer Zunahme der schlechten Vorwärtshaltungszeit;

Anzeigen des zweiten Bereichs dunkler, stärker oder opaker mit einer Zunahme der schlechten Rückwärtshaltungszeit;

Anzeigen des dritten Bereichs dunkler, stärker oder opaker mit einer Zunahme der schlechten Linkshaltungszeit; und

Anzeigen des vierten Bereichs dunkler, stärker oder opaker mit einer Zunahme der schlechten Rechtshaltungszeit.

7. Betriebsverfahren nach Anspruch 3, umfassend:

Erfassen, durch das Benutzerendgerät, von kumulativen täglichen Haltungsinformationen durch Akkumulieren der täglichen Haltungsinformationen in vorbestimmten Intervallen während einer vorbestimmten Sammelzeit;
Erfassen einer Zeit mit täglichen Haltungsinformationen, die angeben, dass sich der Benutzer nach vorne neigt, in den kumulativen täglichen Haltungsinformationen als eine Vorwärtshaltungszeit;
Erfassen einer Zeit mit täglichen Haltungsinformationen, die angeben, dass sich der Benutzer nach hinten neigt, in den kumulativen täglichen Haltungsinformationen als eine Rückwärtshaltungszeit;
Erfassen einer Zeit mit täglichen Haltungsinformationen, die angeben, dass sich der Benutzer nach links neigt, in den kumulativen täglichen Haltungsinformationen als eine Linkshaltungszeit;
Erfassen einer Zeit mit täglichen Haltungsinformationen, die angeben, dass sich der Benutzer nach rechts neigt, in den kumulativen täglichen Haltungsinformationen als eine Rechtshaltungszeit;
Anzeigen des ersten Bereichs dunkler, stärker oder opaker mit einer Zunahme der Vorwärtshaltungszeit;
Anzeigen des zweiten Bereichs dunkler, stärker oder opaker mit einer Zunahme der Rückwärtshaltungszeit;
Anzeigen des dritten Bereichs dunkler, stärker oder opaker mit einer Zunahme der Linkshaltungszeit; und
Anzeigen des vierten Bereichs dunkler, stärker oder opaker mit einer Zunahme der Rechtshaltungszeit.

8. Betriebsverfahren nach Anspruch 1, umfassend:

Erfassen, durch das Benutzerendgerät, von kumulativen täglichen Haltungsinformationen durch Akkumulieren der täglichen Haltungsinformationen in vorbestimmten Intervallen während einer vorbestimmten Sammelzeit;
Erfassen einer Zeit mit täglichen Haltungsinformationen, die in den Referenzbereichsinformationen enthalten sind, in den kumulativen täglichen Haltungsinformationen als eine gute Haltungszeit;
Erfassen einer Zeit mit täglichen Haltungsinformationen, die nicht in den Referenzbereichsinformationen enthalten sind, in den kumulativen täglichen Haltungsinformationen als eine schlechte Haltungszeit;
Erfassen eines Werts, der durch Dividieren der guten Haltungszeit durch die Sammelzeit berechnet wird, als einen Haltungswert; und
Anzeigen des Haltungswertes.

9. Betriebsverfahren nach Anspruch 1, umfassend:

Erfassen, durch mindestens eines von dem Benutzerendgerät und dem Haltungssensor, eines Haltungsmusters des Benutzers; und
Auswählen von mindestens einem von Referenzbereichsinformationen und dem Modell für maschinelles Lernen zur Haltungsschätzung entsprechend dem Haltungsmuster,
wobei das Haltungsmuster mindestens eines von einer stehenden Pose, einer sitzenden Pose, einer gehenden Pose und einer laufenden Pose umfasst.

10. Betriebsverfahren nach Anspruch 1, wobei die 3D-Haltungsinformationen eine Mehrzahl von Körpermerkmalsknoten, die mindestens einigen Gelenken des Körpers entsprechen, und Skelettkanten, die die Mehrzahl von Körpermerkmalsknoten verbinden, umfassen, und
das Erfassen der 3D-Haltungsinformationen ein Bewirken umfasst, dass ein Winkel einer Skelettkante, die in den 3D-Haltungsinformationen enthalten ist und der Wirbelsäule entspricht, in Bezug auf eine Bodenfläche den täglichen Haltungsinformationen entspricht.

11. Betriebsverfahren nach Anspruch 1, umfassend:

Auswählen, durch das Benutzerendgerät, von für den Benutzer vorteilhaften Übungsinformationen aus einer Mehrzahl von Elementen von Kandidatenübungsinformationen auf der Grundlage der täglichen Haltungsinformationen;
Ausgeben, durch das Benutzerendgerät, der Übungsinformationen; und
Erfassen, durch das Benutzerendgerät, der Haltungsbildinformationen des Benutzers auf der Grundlage des Übungshaltungssensors, wenn der Benutzer eine Haltung auf der Grundlage der Übungsinformationen einnimmt.

12. Betriebsverfahren nach Anspruch 11, wobei das Auswählen der Übungsinformationen umfasst:

Erfassen, durch das Benutzerendgerät, von Krankheitsinformationen von dem Benutzer;
Auswählen von Teilkandidatenübungsinformationen durch Ausschließen von für den Benutzer schädlichen Übungsinformationen aus der Mehrzahl von Elementen von Kandidatenübungsinformationen auf der Grundlage der Krankheitsinformationen; und
Auswählen der Übungsinformationen aus den Teilkandidatenübungsinformationen auf der Grundlage der täglichen Haltungsinformationen.

13. Betriebsverfahren nach Anspruch 1, wobei das Erfassen der täglichen Haltungsinformationen umfasst:

Anzeigen eines täglichen Haltungsobjekts, das mindestens einen konzentrischen Kreis um ein Objekt umfasst, das den von oben gesehenen Benutzer darstellt; und
Anzeigen einer Farbe, die den in den täglichen Haltungsinformationen enthaltenen Größeninformationen entspricht, an einer Position, die den in den täglichen Haltungsinformationen enthaltenen Richtungsinformationen entspricht, in dem täglichen Haltungsobjekt.

14. Betriebsverfahren nach Anspruch 1, wobei das Bestimmen, durch das Benutzerendgerät, der Haltungsähnlichkeit zwischen den 3D-Haltungsinformationen und den vorbestimmten Referenzhaltungsinformationen umfasst:

Bestimmen, ob die 3D-Haltungsinformationen guten Haltungsinformationen für jeden vorbestimmten Körperteil des Benutzers entsprechen;
Erhöhen eines Gesundheitswertes eines entsprechenden Teils mit einer Zunahme einer Ähnlichkeit zwischen den 3D-Haltungsinformationen und den guten Haltungsinformationen; und
Anzeigen eines Diagramms, das Gesundheitswerte der Körperteile zeigt, in einem Bereich eines Bildschirms.

**Revendications**

1. Procédé de fonctionnement d'un système de correction de posture destiné à corriger une posture à l'aide de l'intelligence artificielle (IA), dans lequel le système de correction de posture comprend un serveur (210), un terminal utilisateur (100) et un capteur de posture (220), le procédé de fonctionnement comprenant:

la définition, par le terminal utilisateur, d'informations de plage de référence d'une bonne posture à partir d'une entrée d'un utilisateur;
l'acquisition d'informations quotidiennes relatives à la posture de l'utilisateur à l'aide d'un capteur de posture quotidienne qui est intégré à un capteur de posture et qui mesure l'inclinaison de la colonne vertébrale de l'utilisateur;
la sortie, sur le terminal utilisateur, d'un message recommandant de redresser la posture lorsque les informations quotidiennes relatives à la posture ne se situent pas dans les informations de plage de référence;
l'acquisition d'informations relatives à la posture de l'utilisateur à partir d'un capteur de posture d'exercice qui est intégré au capteur de posture et qui capture l'ensemble du corps de l'utilisateur;
l'acquisition, par le serveur, d'informations relatives à la posture en trois dimensions (3D) par applications des informations d'image de posture et des informations de posture quotidiennes à un modèle d'apprentissage automatique d'estimation de posture;
la détermination, par le serveur, de la similitude de posture entre les informations de posture 3D et les informations de posture de référence prédéfinies; et
la sortie, sur le terminal de l'utilisateur, d'un message recommandant de corriger la posture lorsque la similarité de la posture est inférieure à un seuil prédéterminé.

2. Procédé de fonctionnement selon la revendication 1, dans lequel la définition des informations de plage de référence comprend:

la réception d'une entrée utilisateur associée aux informations de plage de référence, en provenance de l'utilisateur;
la détermination, par le terminal utilisateur, des informations de plage initiale prédéterminées en tant qu'informations de plage de référence lorsque l'entrée utilisateur indique une définition automatique de plage;

l'affichage, par le terminal utilisateur, d'une interface utilisateur pour la définition de la plage lorsque l'entrée utilisateur indique une définition manuelle de la plage;

l'acquisition, par le terminal utilisateur, la mesure, de l'inclinaison de la colonne vertébrale de l'utilisateur à partir des données du capteur de posture quotidienne;

la modification, par le terminal utilisateur, de l'inclinaison d'un objet représentant au moins une partie du corps, intégré à l'interface utilisateur, en fonction de l'inclinaison de la colonne vertébrale, et l'affichage de l'objet; et

la détermination, par le terminal utilisateur, d'une inclinaison de la colonne vertébrale de 0 degré comme informations de plage de référence lorsque l'utilisateur confirme l'inclinaison de son corps et saisit un objet représentant cette confirmation.

3. Procédé de fonctionnement selon la revendication 1, dans lequel l'acquisition des informations de posture quotidienne comprend

l'affichage d'un objet de posture quotidienne comprenant une première zone, une deuxième zone, une troisième zone et une quatrième zone; et

l'affichage au moins d'une zone parmi les première, deuxième, troisième, quatrième zones dans une couleur prédéterminée en fonction des informations quotidiennes relatives à la posture.

4. Procédé selon la revendication 3, dans lequel l'affichage de l'au moins une parmi la première, deuxième, troisième, quatrième zones dans la couleur prédéterminée comprend:

l'affichage de la première zone dans la couleur prédéterminée lorsque les informations de posture quotidienne indiquent que l'utilisateur se penche vers l'avant;

l'affichage de la deuxième zone dans la couleur prédéterminée lorsque les informations de posture quotidienne indiquent que l'utilisateur se penche en arrière;

l'affichage de la troisième zone dans la couleur prédéterminée lorsque les informations de posture quotidienne indiquent que l'utilisateur se penche vers la gauche;

l'affichage de la quatrième zone dans la couleur prédéterminée lorsque les informations de posture quotidienne indiquent que l'utilisateur se penche vers la droite.

5. Procédé selon la revendication 3, dans lequel la première région comprend une région 1-1 et une région 1-2,

la deuxième région comprend une région 2-1 et une région 2-2,

la troisième région comprend une région 3-1 et une région 3-2,

la quatrième région comprend une région 4-1 et une région 4-2, et

le procédé de fonctionnement comprend en outre:

l'affichage de la zone 1-1 dans la couleur prédéterminée lorsque les informations de posture quotidienne indiquent que l'utilisateur se penche vers l'avant et que les informations relatives à l'amplitude d'inclinaison, contenues dans les informations de posture quotidienne, se situent dans la plage de référence;

l'affichage de la zone 1-2 dans la couleur prédéterminée lorsque les informations de posture quotidienne indiquent que l'utilisateur se penche vers l'avant et que les informations relatives à l'amplitude d'inclinaison, contenues dans les informations de posture quotidienne, ne se situent pas dans la plage de référence;

l'affichage de la zone 2-1 dans la couleur prédéterminée lorsque les informations de posture quotidienne indiquent que l'utilisateur se penche en arrière et que les informations relatives à l'amplitude d'inclinaison, contenues dans les informations de posture quotidienne, se situent dans la plage de référence;

l'affichage de la zone 2-2 dans la couleur prédéterminée lorsque les informations de posture quotidienne indiquent que l'utilisateur se penche en arrière et que les données relatives à l'amplitude d'inclinaison, contenues dans les informations de posture quotidienne, ne se situent pas dans la plage de référence;

l'affichage de la zone 3-1 dans la couleur prédéterminée lorsque les informations de posture quotidienne indiquent que l'utilisateur se penche vers la gauche et que les données relatives à l'amplitude d'inclinaison, contenues dans les informations de posture quotidienne, se situent dans la plage de référence;

l'affichage de la zone 3-2 dans la couleur prédéterminée lorsque les informations de posture quotidienne indiquent que l'utilisateur se penche vers la gauche et que les informations relatives à l'amplitude d'inclinaison, contenues dans les informations de posture quotidienne, ne se situent pas dans la plage de référence;

l'affichage de la zone 4-1 dans la couleur prédéterminée lorsque les informations de posture quotidienne indiquent que l'utilisateur se penche en la droite et que les informations relatives à l'amplitude d'inclinaison, contenues dans les informations de posture quotidienne, se situent dans la plage de référence; et

l'affichage de la zone 4-2 dans la couleur prédéterminée lorsque les informations de posture quotidienne indiquent que l'utilisateur se penche vers la droite et que les informations relatives à l'amplitude d'inclinaison, contenues dans les informations de posture quotidienne, ne se situent pas dans la plage de référence.

6. Procédé de fonctionnement selon la revendication 3, comprenant:

l'acquisition, par le terminal utilisateur, d'informations cumulées relatives à la posture quotidienne, en cumulant les informations quotidiennes relatives à la posture à des intervalles prédéterminés pendant une période de collecte prédéterminée;

l'acquisition d'une durée avec des informations de posture quotidienne, qui n'est pas comprise dans les informations de plage de référence et indique que l'utilisateur se penche vers l'avant, dans les informations cumulées relatives à la posture quotidienne en tant que période de mauvaise posture vers l'avant;

l'acquisition d'une durée avec des informations de posture quotidienne, qui n'est pas comprise dans les informations de plage de référence et indique que l'utilisateur se penche en arrière, dans les informations cumulées relatives à la posture quotidienne, en tant que période de mauvaise posture en arrière;

l'acquisition d'une durée avec des informations de posture quotidienne, qui n'est pas comprise dans les informations de plage de référence et indique que l'utilisateur se penche vers la gauche, dans les informations relatives à la posture quotidienne en tant que période de mauvaise posture vers la gauche;

l'acquisition d'une durée avec des informations de posture quotidienne, qui n'est pas comprise dans les informations de plage de référence et indique que l'utilisateur se penche vers la droite, dans les informations cumulées de posture quotidienne en tant que période de mauvaise posture vers la droite;

l'affichage de la première zone de manière plus sombre, plus intense ou plus opaque à mesure que la durée de la mauvaise posture vers l'avant augmente;

l'affichage de la deuxième zone de manière plus sombre, plus intense ou plus opaque à mesure que la durée de la mauvaise posture en arrière augmente;

l'affichage de la troisième zone de manière plus sombre, plus intense ou plus opaque à mesure que la durée de la mauvaise posture vers la gauche augmente;

l'affichage de la quatrième zone de manière plus sombre, plus intense ou plus opaque à mesure que la durée de la mauvaise posture vers la droite augmente;

7. Procédé de fonctionnement selon la revendication 3, comprenant:

l'acquisition, par le terminal utilisateur, d'informations cumulées relatives à la posture quotidienne, en cumulant les informations quotidiennes relatives à la posture à des intervalles prédéterminés pendant une période de collecte prédéterminée;

l'acquisition d'une durée avec des informations de posture quotidienne, qui indique que l'utilisateur se penche vers l'avant, dans les informations cumulées de poste quotidienne en tant que période de posture vers l'avant;

l'acquisition d'une durée avec des informations de posture quotidienne, qui indique que l'utilisateur se penche en arrière, dans les informations cumulées de posture quotidienne en tant que période de posture en arrière;

l'acquisition d'une durée avec des informations de posture quotidienne, qui indique que l'utilisateur se penche vers la gauche, dans les informations cumulées de posture quotidienne en tant que période de posture vers la gauche;

l'acquisition d'une durée avec des informations de posture quotidienne, qui indique que l'utilisateur se penche vers l'avant, dans les informations cumulées de posture quotidienne en tant que période de posture vers la droite;

l'affichage de la première zone de manière plus sombre, plus intense ou plus opaque à mesure que la durée de la posture vers l'avant augmente;

l'affichage de la deuxième zone de manière plus sombre, plus intense ou plus opaque à mesure que la durée de la posture en arrière augmente;

l'affichage de la troisième zone de manière plus sombre, plus intense ou plus opaque à mesure que la durée de la posture vers la gauche augmente; et

l'affichage de la quatrième zone de manière plus sombre, plus intense ou plus opaque à mesure que la durée de la posture vers la droite augmente;

8. Procédé de fonctionnement selon la revendication 1, comprenant:

l'acquisition, par le terminal utilisateur, d'informations cumulées relatives à la posture quotidienne, en cumulant les informations quotidiennes relatives à la posture à des intervalles prédéterminés pendant une période de collecte prédéterminée;

l'acquisition d'une durée avec des informations de posture quotidienne, qui sont comprises dans les informations de plage de référence, dans les informations cumulées de posture quotidienne, en tant que période de bonne posture;

l'acquisition d'une durée avec des informations de posture quotidienne, qui ne sont pas comprises dans les informations de plage de référence, dans les informations cumulées de posture quotidienne en tant que période de mauvaise posture;

l'acquisition d'une valeur calculée par division de la durée de bonne posture par la durée totale qui sert de score de posture; et

l'affichage du score de posture.

9. Procédé de fonctionnement selon la revendication 1, comprenant:

l'acquisition, par au moins l'un parmi le terminal utilisateur et le capteur de posture, un modèle de posture de l'utilisateur; et

la sélection d'au moins un élément parmi les informations relatives à la plage de référence et le modèle d'apprentissage automatique d'estimation de la posture correspondant au type de posture,

dans lequel le type de posture comprend au moins l'une parmi une posture debout, une posture assise, une posture de marche et une posture de course.

10. Procédé de fonctionnement selon la revendication 1, dans lequel les informations de posture 3D comprennent une pluralité de nœuds caractéristiques du corps qui correspondent à au moins certaines articulations du corps, ainsi que des contours squelettiques reliant cette pluralité de nœuds caractéristiques du corps, et

l'acquisition des informations de posture en 3D consiste à faire en sorte que l'angle d'un contour squelettique, qui est inclus dans les informations de posture en 3D et correspond à la colonne vertébrale, par rapport à une surface au sol, corresponde aux informations de posture quotidienne.

11. Procédé de fonctionnement selon la revendication 1, comprenant:

la sélection, par le terminal utilisateur, d'informations d'exercices utiles à l'utilisateur parmi une pluralité d'informations d'exercices candidates, sur la base des informations relatives à la posture quotidienne;

la sortie, par le terminal utilisateur, des informations d'exercices; et

l'acquisition, par le terminal utilisateur, des informations d'image de posture de l'utilisateur à partir du capteur de posture d'exercice lorsque l'utilisateur adopte une posture conformément aux informations d'exercice.

12. Procédé de fonctionnement selon la revendication 11, dans lequel la sélection des informations d'exercice comprend:

l'acquisition, par le terminal de l'utilisateur, d'informations relatives à la maladie fournies par l'utilisateur;

la sélection d'informations partielles sur les exercices candidats en excluant, parmi l'ensemble des informations sur les exercices candidats, celles qui pourraient nuire à l'utilisateur, sur la base des informations relatives à la maladie; et

la sélection des informations d'exercice parmi les informations partielles sur les exercices candidats, en se basant sur les informations relatives à la posture quotidienne.

13. Procédé de fonctionnement selon la revendication 1, dans lequel l'acquisition des informations de posture quotidienne comprend:

l'affichage d'un objet représentant la posture quotidienne, comprenant au moins un cercle concentrique autour d'un objet représentant l'utilisateur vu d'en haut; et

l'affichage d'une couleur correspondant aux informations d'intensité contenues dans les informations de posture quotidienne, à un emplacement correspondant aux informations de direction contenues dans les informations de posture quotidienne, au sein de l'objet de posture quotidienne.

14. Procédé de fonctionnement selon la revendication 1, dans lequel la détermination, par le terminal utilisateur, de la similarité de posture entre les informations de posture 3D et les informations de posture de référence prédéterminées comprend:

la détermination visant à savoir si les informations de posture en 3D correspondent à une bonne posture pour chaque partie du corps prédéfinie de l'utilisateur;

l'augmentation du score de santé d'une partie correspondante à mesure que la similarité entre les informations de posture 3D et les informations de bonne posture augmente; et

l'affichage d'un graphique présentant les scores de santé des différentes parties du corps dans une zone de l'écran.

FIG. 1

FIG. 2

FIG. 3

```
                        ┌─────────────┐
                        │    START    │
                        └──────┬──────┘
                               ↓
        ┌──────────────────────────────────────────────┐  ⌐ 310
        │  SET REFERENCE RANGE INFORMATION OF GOOD POSTURE │
        └──────────────────────┬───────────────────────┘
                               ↓
        ┌──────────────────────────────────────────────┐  ⌐ 320
        │     ACQUIRE DAILY POSTURE INFORMATION OF USER    │
        └──────────────────────┬───────────────────────┘
                               ↓
        ┌──────────────────────────────────────────────┐  ⌐ 330
        │ OUTPUT MESSAGE RECOMMENDING STRAIGHTENING UP POSTURE │
        └──────────────────────┬───────────────────────┘
                               ↓
        ┌──────────────────────────────────────────────┐  ⌐ 340
        │    ACQUIRE POSTURE IMAGE INFORMATION OF USER     │
        └──────────────────────┬───────────────────────┘
                               ↓
        ┌──────────────────────────────────────────────┐  ⌐ 350
        │          ACQUIRE 3D POSTURE INFORMATION          │
        └──────────────────────┬───────────────────────┘
                               ↓
        ┌──────────────────────────────────────────────┐  ⌐ 360
        │          DETERMINE POSTURE SIMILARITY            │
        └──────────────────────┬───────────────────────┘
                               ↓
        ┌──────────────────────────────────────────────┐  ⌐ 370
        │ OUTPUT MESSAGE RECOMMENDING CORRECTING POSTURE   │
        └──────────────────────┬───────────────────────┘
                               ↓
                        ┌─────────────┐
                        │     END     │
                        └─────────────┘
```

FIG. 4

|  | FRONT | BACK | LEFT | RIGHT |
|---|---|---|---|---|
| AUTOMATIC RANGE SETTING | 20° | 15° | 10° | 10° |
| MANUAL RANGE SETTING | 0°~30° | 0°~30° | 0°~30° | 0°~30° |

FIG. 5

FIG. 6

FIG. 7

FIG. 8

FIG. 9

FIG. 10

FIG. 11

FIG. 12

FIG. 13

FIG. 14

FIG. 15

FIG. 16

```
                    ┌─────────────┐
                    │    START    │
                    └──────┬──────┘
                           │
                           ▼
   ┌──────────────────────────────────────────────────┐        1610
   │  ACQUIRE CUMULATIVE DAILY POSTURE INFORMATION     │
   └──────────────────────┬───────────────────────────┘
                           │
                           ▼
   ┌──────────────────────────────────────────────────┐        1620
   │           ACQUIRE GOOD POSTURE TIME              │
   └──────────────────────┬───────────────────────────┘
                           │
                           ▼
   ┌──────────────────────────────────────────────────┐        1630
   │           ACQUIRE BAD POSTURE TIME               │
   └──────────────────────┬───────────────────────────┘
                           │
                           ▼
   ┌──────────────────────────────────────────────────┐        1640
   │           ACQUIRE POSTURE SCORE                  │
   └──────────────────────┬───────────────────────────┘
                           │
                           ▼
   ┌──────────────────────────────────────────────────┐        1650
   │           DISPLAY POSTURE SCORE                  │
   └──────────────────────┬───────────────────────────┘
                           │
                           ▼
                    ┌─────────────┐
                    │     END     │
                    └─────────────┘
```

FIG. 17

1700

WEEKLY ACTIVITY

MAR 6 TO MAR 12 (2022)

DAILY AVERAGE

GOOD POSTURE
46 MINUTES
INCREASED BY 46 MINUTES
FROM LAST WEEK

POSTURE SCORE
37
INCREASED BY 25 POINTS
FROM LAST WEEK

1720

1710

GOOD
38/39 MINIUTES
THU, MAR 10

200MINUTES

100MINUTES

1730

0MINUTES

SUN     MON     TUE     WED     THU     FRI     SAT

● GOAL   ● GOOD POSTURE DURATION

FIG. 18

FIG. 19

FIG. 20

## AI ANALYSIS RESULTS

TORSO

LEFT ARM        RIGHT ARM

LEFT LEG        RIGHT LEG

2010

| CERVICAL SPINE BALANCE | THORACIC SPINE BALANCE | LUMBAR SPINE BALANCE |
|---|---|---|
| 70 POINTS | 60 POINTS | 40 POINTS |

2020

MY SPINAL HEALTH ANALYSIS RESULT

EXPECTED HERNIATED DISK SCORE 55 POINTS

2030

SEE CUSTOMIZED EXERCISE

2040

**REFERENCES CITED IN THE DESCRIPTION**

**Patent documents cited in the description**

- KR 101480026
- KR 20210064999 A
- CN 116310083 A
- KR 102593121 B1